(19) Europäisches Patentamt
European Patent Office
Office européen des brevets

(11) **EP 2 897 523 B1**

(12) **EUROPEAN PATENT SPECIFICATION**

(45) Date of publication and mention
of the grant of the patent:
**22.05.2024   Bulletin 2024/21**

(21) Application number: **13838364.1**

(22) Date of filing: **19.09.2013**

(51) International Patent Classification (IPC):
*G01R 33/46* (2006.01)    *G01R 33/465* (2006.01)
*G01R 33/54* (2006.01)    *G01R 33/561* (2006.01)
*G01R 33/48* (2006.01)    *G01R 33/50* (2006.01)
*G01R 33/563* (2006.01)    *G01R 33/44* (2006.01)
*G01R 33/24* (2006.01)

(52) Cooperative Patent Classification (CPC):
**G01R 33/561; G01R 33/4616; G01R 33/4625;
G01R 33/465; G01R 33/543;** G01R 33/24;
G01R 33/448; G01R 33/4828; G01R 33/50;
G01R 33/56341

(86) International application number:
**PCT/US2013/060681**

(87) International publication number:
**WO 2014/047326 (27.03.2014 Gazette 2014/13)**

(54) **NUCLEAR MAGNETIC RESONANCE (NMR) FINGERPRINTING**

NMR-FINGERABDRUCKNAHME

PRISE D'EMPREINTE PAR RÉSONANCE MAGNÉTIQUE NUCLÉAIRE (RMN)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **19.09.2012   US 201213623104**

(43) Date of publication of application:
**29.07.2015   Bulletin 2015/31**

(73) Proprietor: **Case Western Reserve University
Cleveland, OH 44114 (US)**

(72) Inventors:
• **GRISWOLD, Mark**
  **Shaker Heights, OH 44120 (US)**
• **SEIBERLICH, Nicole**
  **Cleveland, OH 44114 (US)**
• **GULANI, Vikas**
  **Cleveland, OH 44106 (US)**
• **MA, Dan**
  **Cleveland, OH 44106 (US)**

(74) Representative: **Samson & Partner Patentanwälte
mbB
Widenmayerstraße 6
80538 München (DE)**

(56) References cited:
**US-A1- 2004 189 296     US-A1- 2005 060 112
US-A1- 2008 309 337     US-A1- 2009 232 410
US-A1- 2010 321 017**

• **JAMES S MCKENZIE ET AL: "Analysis of complex
mixtures using high-resolution nuclear magnetic
resonance spectroscopy and chemometrics",
PROGRESS IN NUCLEAR MAGNETIC
RESONANCE SPECTROSCOPY, PERGAMON
PRESS, OXFORD, GB, vol. 59, no. 4, 27 April 2011
(2011-04-27) , pages 336-359, XP028325834,
ISSN: 0079-6565, DOI:
10.1016/J.PNMRS.2011.04.003 [retrieved on
2011-05-12]**
• **DAN MA ET AL: "Magnetic resonance
fingerprinting", NATURE, vol. 495, no. 7440, 13
March 2013 (2013-03-13), pages 187-192,
XP055183037, ISSN: 0028-0836, DOI:
10.1038/nature11971**

- MARIYA DONEVA ET AL: "Compressed sensing reconstruction for magnetic resonance parameter mapping", MAGNETIC RESONANCE IN MEDICINE, vol. 64, no. 4, 17 June 2010 (2010-06-17), pages 1114-1120, XP055188361, ISSN: 0740-3194, DOI: 10.1002/mrm.22483
- DONALD B. TWIEG: "Parsing local signal evolution directly from a single-shot MRI signal: A new approach for fMRI", MAGNETIC RESONANCE IN MEDICINE, vol. 50, no. 5, 1 November 2003 (2003-11-01), pages 1043-1052, XP055188638, ISSN: 0740-3194, DOI: 10.1002/mrm.10613
- DAN MA ET AL: "MR Fingerprinting (MRF): a Novel Quantitative Approach to MRI", PROCEEDINGS OF THE INTERNATIONAL SOCIETY FOR MAGNETIC RESONANCE IN MEDICINE, 20TH ANNUAL MEETING AND EXHIBITION, MELBOURNE, AUSTRALIA, 5-11 MAY 2012, vol. 20, 21 April 2012 (2012-04-21), page 288, XP055207010,

**Description**

**BACKGROUND**

Conventional magnetic resonance (MR) pulse sequences include a preparation phase, a waiting phase, and an acquisition phase that are configured to produce signals from which images can be made. The preparation phase determines when a signal can be acquired and determines the properties of the acquired signal. For example, a first pulse sequence may be designed to produce a T1-weighted signal at a first echo time (TE) while a second pulse sequence may be designed to produce a T2-weighted signal at a second TE. However, a lot of preparations and a lot of short waits, especially when compounded over multiple pulse sequences, can add up to a long time to collect a data set. These conventional pulse sequences are typically designed to provide qualitative results where data are acquired with various weightings or contrasts that highlight a particular parameter (e.g., T1 relaxation, T2 relaxation).

A conventional MR acquisition involves numerous repetitions of prepare/wait/acquire pulse sequences. For example, the first pulse sequence may be applied a large number of times to acquire TI weighted signals for all voxels in a volume of interest (RoI) and then the second pulse sequence may be applied a large number of times to acquire T2 weighted signals for all the voxels in the RoI. Registering (e.g., aligning) the signals from these two acquisitions may be difficult.

When MR images are generated, they may be viewed by a radiologist and/or surgeon who interprets the qualitative images for specific disease signatures. The radiologist may examine multiple image types (e.g., T1-weighted, T2-weighted) acquired in multiple imaging planes to make a diagnosis. The radiologist or other individual examining the qualitative images may need particular skill to be able to assess changes from session to session, from machine to machine, and from machine configuration to machine configuration. Thus, the images are only as good as the image interpreter and all image based (e.g., qualitative) diagnoses end up being subjective.

Seen from a different point of view, conventional MR uses precise preparation time to create precise preparation conditions that facilitate acquiring precise signals from precise locations at precise points in time to make *imprecise* qualitative data sets. Conventional MR attempts to force the scanned contents (e.g., water, fat) to emit certain signals at certain times and then reconstructs data from these signals. Regardless of these shortcomings, conventional MR has served the clinical community well for many years.

Twieg proposed an approach involving compressed sensing where a model of a signal was used to reduce the total amount of data needed to reconstruct a parameter map and then to reconstruct an image. Similarly, Doneva et al. proposed random under-sampling to achieve compressed sensing. In the Doneva approach, a pixel will represent its true signal evolution plus aliased signal from other pixels. In one embodiment, the aliasing will only appear as added noise at a pixel. The noise will not have structure and will not correlate to the true signal evolution. The Doneva approach facilitates performing a relatively simple process like Orthogonal Matching Pursuit (OMP) to resolve the correct signal to support image reconstruction. OMP assumes the presence of a constrained dictionary of expected signal evolutions. OMP compares a received signal to the dictionary of signals to identify the signal that was most likely to come from a pixel.

Twieg, Parsing local signal evolution directly from a single-shot MRI signal: a new approach for fMRI, Magn Reson Med 2003, Nov; 50(5):1043-52, describes a single-shot MRI method that performs single-shot parameter assessment by retrieval from signal encoding. The Twieg method abandons the fundamental simplifying assumption used in conventional MRI methods, that the local intrinsic signal does not change its amplitude or phase during signal acquisition, even though these changes may be substantial, especially during longer periods used in single-shot image acquisitions. Twieg recognized that local decay and phase evolution occur and therefore modeled each signal datum as a sample from (k, t) space rather than k-space. Twieg adopted the view that each datum has its own location in a (k, t) space that also reflects another attribute (e.g., relaxation, decay), where t is the elapsed time. While Twieg anticipated improved accuracy and robustness due to the new signal model, intensive reconstruction computations limited Twieg's progress.

Doneva, et al., Compressed sensing reconstruction for magnetic resonance parameter mapping, Magnetic Resonance in Medicine, Volume 64, Issue 4, pages 1114-1120, October 2010, recognizes that different tissues in the human body can be distinguished in MRI by their intrinsic MR parameters including proton density, longitudinal (T1, spin-lattice) relaxation time, and transverse (T2, spin-spin) relaxation time. Doneva applies a learned dictionary to sparsify data and then uses a model based reconstruction for MR parameter mapping. Doneva identifies that "multiple relaxation components in a heterogeneous voxel can be assessed." However, Doneva uses an imaging based approach that relies on a library whose curves can, in one example, be characterized by equations of the form:

$$SE = 1 - 2e^{-t/Tx}$$

where:

SE is a signal evolution,

t is time, and

Tx is a single relaxation parameter.

In another, more general example, Doneva uses an imaging based approach that relies on a library whose curves can be characterized by:

$$SE = A + Be^{-t/C}$$

where A is a constant, B is a constant, t is time, and C is a single relaxation parameter.

Doneva pattern matches a received signal evolution to a curve stored in the library.

The Doneva library is limited to the idealized, single relaxation parameter curves because the preparation is specific and constrained by the fact that Doneva ultimately reconstructs an image from the acquired data. Thus, any variations in t appear to be constant or linear and any variations in $\alpha$ also appear to be constant or linear.

Twieg and Doneva appear to be limited to conventional imaging sequences that highlight only one or a few parameters. To the extent that Twieg or Doneva use any quantitative sequences, these sequences include an excitation and preparation scheme that generates a contrast between different tissues with different properties. However, the preparation fades over time until no more useful information can be acquired unless preparation is repeated. For example, after about 4-5 seconds, tissues subjected to an inversion recovery sequence designed for T1 contrast will have recovered to their equilibrium state and will yield no more signal. This short time limit compromises the ability to perform three dimensional imaging, imaging of moving targets, and so on. Additionally, Twieg and Doneva appear further limited to acquiring information, associated with one relaxation parameter at a time. Twieg and Doneva appear suited to collecting information about T1 relaxation, T2 relaxation, or one fixed combination of T1 and T2, but not both simultaneously. To the extent that Twieg and Doneva could acquire information about T1 and T2, the sensitivity to either would be constant through the acquisition.

James S McKenzie et al. discloses "Analysis of complex mixtures using high-resolution nuclear magnetic resonance spectroscopy and chemometrics", Progress in Nuclear Magnetic Resonance Spectroscopy vol. 59, no. 4, 27 April 2011. Dan Ma et al. discloses in "MR Fingerprinting (MRF): a Novel Quantitative Approach to MRI", Proceedings of the International Society for Magnetic Resonance in Medicine, vol. 20, page 288 (2012) an early version of magnetic resonance fingerprinting, wherein the flip angle and repetition time are randomly varied during acquisition to generate a different signal evolution for different tissues having different relaxation parameters, and a pattern recognition based reconstruction is used to derive quantitative estimates of underlying relaxation parameters.

## BRIEF DESCRIPTION OF THE DRAWINGS

The accompanying drawings, which are incorporated in and constitute a part of the specification, illustrate various example systems, methods, and other example embodiments of various aspects of the invention. It will be appreciated that the illustrated element boundaries (e.g., boxes, groups of boxes, or other shapes) in the figures represent one example of the boundaries. One of ordinary skill in the art will appreciate that in some examples one element may be designed as multiple elements or that multiple elements may be designed as one element. In some examples, an element shown as an internal component of another element may be implemented as an external component and vice versa. Furthermore, elements may not be drawn to scale.

FIG. 1 illustrates a volume that contains two resonant species.

FIG. 2 illustrates two individual NMR signals received from two resonant species and a signal evolution derived from the two individual NMR signals.

FIG. 3 compares and contrasts conventional sequence blocks to example sequence blocks.

FIG. 4 illustrates an example method associated, with NMR fingerprinting.

FIG. 5 illustrates an example method associated with NMR fingerprinting.

FIG. 6 illustrates an example apparatus associated with NMR fingerprinting.

FIG. 7 illustrates an example apparatus associated with NMR fingerprinting.

FIG. 8 illustrates an MR apparatus configured to perform NMR fingerprinting.

FIG. 9 illustrates another example set of sequence blocks.

FIGS. 10a-10b illustrate an example magnetic resonance fingerprinting (MRF) sequence pattern. FIG. 10a illustrates an example acquisition sequence diagram. FIG. 10b illustrates an example of FA and TR patterns.

Figures 11a-11c illustrate example signal properties and matching results. Figure I1a illustrates simulated signal evolution curves corresponding to four normal tissues of the brain. Figure 11b illustrates one example of acquired signal evolution curves and its comparison to a dictionary. Figure 11c illustrates T1 and T2 values retrieved from a matching algorithm.

Figures 12a-12d illustrate example in vivo results associated with NMR fingerprinting. Figure 12a illustrates a T1 map (ms), Figure 12b illustrates a T2 map (ms), Figure 12c illustrates an off-resonance map (Hz), and Figure 12d illustrates a proton density map retrieved.

Figure 13 illustrates an apparatus configured to compare acquired information to reference information.

## DETAILED DESCRIPTION

The invention is set out in the appended set of claims. Example apparatus and methods employ a series of varied sequence blocks that produce different signal evolutions in different resonant species (e.g., tissues) to which the RF is applied. The term "resonant species", as used herein, refers to an item (e.g., water, fat, tissue, material) that can be made to resonate using NMR. By way of illustration, when example apparatus and methods apply RF energy to a volume that has both bone and muscle tissue, then both the bone and muscle tissue will produce an NMR signal. However the "bone signal" and the "muscle signal" will be different. The different signals can be collected over a period of time to identify a signal evolution for the volume. In one embodiment, resonant species in the volume can then be characterized by comparing the signal evolution to known evolutions. In one embodiment, the "known" evolutions may be, for example, simulated evolutions and/or previously acquired evolutions. Characterizing the resonant species can include identifying different properties of a resonant species (e.g., Tl, T2, diffusion resonant frequency, diffusion co-efficient, spin density, proton density). Additionally, other properties including, but not limited to, tissue types, materials, superposition of attributes (e.g., Tl, T2) can be identified.

According to the invention, characterizing the resonant species is performed by comparing first information to second information. The first information may include the acquired NMR signals, or the acquired signal evolution. The second information may include a stored signal evolution, a known signal evolution, a modeled signal

Comparing the first information to the second information may be performed in various ways including, but not limited to, pattern matching, selection, minimization of a cost function, and optimization. Pattern matching may include, but is not limited to, OMP, categorical sequence labeling, regression, clustering, classification, real value sequence labeling, parsing algorithms, Bayesian methods, Markov methods, ensemble learning methods, and template matching. Optimization may include, but is not limited to, least squares optimization, regularized least squares optimization, basis pursuit optimization, and matching pursuit optimization.

The result of the comparison may take different forms. In different embodiments, the result of the comparison may include, but is not limited to, an identification that the first information matches the second information, an identification that the first information matches the second information to within a tolerance, and an identification that there is a certain percent likelihood that the first information matches the second information. In other embodiments the result of the comparison may include, but is not limited to, an identification of T1 for a resonant species, an identification of T2 for a resonant species, an identification of a diffusion coefficient, an identification of a spin density, an identification of a resonance frequency (e.g., chemical shift) and an identification of a proton density. In another embodiment, the comparison may include identifying the strength of a magnetic field (e.g., B0, B1) or may include identifying the strength of a gradient field. In yet another embodiment, the result of the comparison may identify a tissue type (e.g., brain, brain tumor) or may identify a material. Thus, the comparison may produce different results. In one embodiment, multiple results may be provided. For example, a weighted list of likely materials may be provided. In another example, multiple probabilities may be provided.

Example apparatus and methods do not define what the signals produced by the resonant species must be, only that the signals be different between different resonant species being examined. Unlike conventional systems, the different NMR signals may not have constant signal strength or phase. Since tissues or other materials may produce different signals, in one embodiment, the process of characterizing the tissues or other materials is reduced to pattern recognition in the signal time course. The pattern recognition may be performed using, for example, different variations of Orthogonal Matching Pursuit (OMP). In one embodiment, pattern matching may lead to a conclusion that an acquired signal evolution matches a known signal evolution to within a desired tolerance. In another embodiment, pattern matching may identify a probability that a known signal evolution matches the acquired signal evolution. In another embodiment, comparing may lead to a conclusion that an acquired signal evolution or information that is a function of an acquired signal evolution matches a known signal evolution or information that is a function of one or more known signal evolutions to within a desired tolerance. In yet another embodiment, comparing may identify a probability that an acquired signal evolution or information that is a function of an acquired signal evolution matches a known signal evolution or information that is a function of one or more known signal evolutions. Example apparatus and methods facilitate maximizing contrast between resonant species without ignoring resonant species that may be in the volume or object. Thus, NMR fingerprinting involves applying a series of varied sequence blocks that generates a particular signal evolution signature (e.g., fingerprint) that is specific for a particular combination of parameters and resonant species in a volume. Processing performed on received signals does not involve conventional reconstruction, but rather involves analyzing the received NMR signals or determined signal evolution in light of known information including, but not limited to, signal evolutions, information derived from signal evolutions, and other information.

Larger objects like human bodies are made up of smaller objects like arms and legs and hips. The smaller objects are in turn made up of smaller parts like skin, muscle, fat, bone, tendon, and prosthetics. These smaller parts are in turn made up of even smaller things like water and minerals. The water and minerals are themselves made up of even smaller things (e.g., hydrogen, oxygen) which in turn are made up of even smaller things (e.g., electrons orbiting a nucleus). The nucleus may include a proton that exhibits "spin". A human body has a large number of protons and thus a large number of spins.

In the presence of a magnetic field, some of the spins will align in one direction (e.g., N/S) with respect to that magnetic field while other spins will align in an opposite direction (e.g., S/N) with respect to that magnetic field. Conventional MRI manipulates the magnetic field so that a net alignment in one direction is achieved. Conventional MRI further manipulates the magnetic field so that local differences in the field are achieved to allow spatial encoding. For example, x, y, and z gradients may be applied to create local variations in the larger magnetic field. The local variations allow the excitation of some spins without the excitation of other spins. Selective excitation is possible because of the Larmor relationship between magnetic fields and spins. The Larmor relationship describes how the frequency at which spins accept RF energy is related to the magnetic field in which the spins are located.

With the local variations created, RF energy may be applied to selected sets of spins associated with a local variation to make those spins behave in a certain way. For example, spins may be forced into a high energy state and forced away from their default alignment. When the RF energy is removed, the spins may return or may be forced to return to their default alignment. Different spins may return to their default alignment at different rates. Similarly, spins may return to their default alignment for different reasons. As the spins return from the forced alignment to the natural alignment, the spins produce a signal that can be detected for a short period of time. Conventional systems are limited by this short period of time and must, therefore, constantly repeat the process that tips the spins out of one alignment and into another alignment from which they can return and produce signal.

Like conventional MR, NMR fingerprinting manipulates the magnetic field and manipulates the application of RF energy at different frequencies. However, example apparatus and methods use a comprehensive inquisitive signal acquisition approach. In one embodiment, NMR fingerprinting employs pseudo-random routines that allow a volume to produce the signal(s) the volume is going to produce in response to a variety of changing conditions created by a variety of changing applications of RF energy. According to the invention, NMR fingerprinting then compares a signal that evolves from the received signals to known signals received from other acquisitions at other times under similar conditions or to a set of simulated expected or predicted curves. If the received signal evolution matches or can be fit to within a threshold of a known, simulated, or predicted signal evolution, then the volume that generated the signal evolution likely holds the same number, type, and mixture of spins as the volume that produced that matched or fitted signal evolution. If material or tissue properties are available for the fitted or matched signal evolution, then conventional property determinations may be skipped. More generally, first information associated with NMR signals acquired in response to NMR fingerprinting excitation is compared to second information associated with reference responses to NMR fingerprinting excitation to characterize a property of a material subjected to the NMR fingerprinting excitation.

The frequency at which water in a volume will accept RF energy is determined by the magnetic field in which the water is located. The frequency can be computed when the magnetic field is known. The frequency at which fat in the same volume will accept RF energy is also determined by the magnetic field in which the fat is located. This frequency can also be computed when the magnetic field is known. Thus, applying multiple frequencies can induce multiple resonant species to resonate. Applying the multiple frequencies under a series of different conditions at different times can cause the resonant species to resonate in different ways. Additionally, applying the multiple frequencies under different conditions at different times can cause the resonant species to resonate and relax in different ways. The different resonations and different relaxations may yield a unique signal evolution for a combination of resonant species. Since the frequency is determined by the magnetic field, the magnetic field may be determined when the frequency is known by analyzing received signal in light of reference signals.

If a volume only has water, then the volume will only produce one signal. If the volume only has fat, then the volume will also only produce one signal, but it will be a different signal. Different amounts of fat and water in the same volume will yield different signals. The combination of signals acquired under different conditions may yield nearly infinitely unique signal evolutions. While the human body is a complicated thing, from a certain point of view it is not that complicated. Every volume in a human body can only hold a finite set of things arranged in a finite set of ways. Over time, a comprehensive library of reference information including, but not limited to, signal evolutions associated with many of the most relevant combinations of resonant species may be acquired and be available to NMR fingerprinting apparatus. The library may store signals that may be referred to as baseline signatures or known signal evolutions. In different embodiments, the library may store simulated and/or predicted signal evolutions. Thus in different examples, "known" signal evolutions may include previously acquired signal evolutions and/or simulated signal evolutions. Additionally, a dictionary or other reference store may include information that is a function of a signal evolution. For example, two signal evolutions may be combined into a different piece of information. Similarly, a single signal evolution may be transformed into a different piece of information. Both signal evolutions and information derived from, computed from, or that is otherwise a function of a signal evolution may be stored. Additionally, in one embodiment, a dictionary or other reference store may include information that did not start as a signal evolution or that is not derived from a signal evolution.

In one embodiment, baseline signatures can be associated with materials that were analyzed solely for producing baseline signatures. For example, a beaker of water may be analyzed for a period of time using varied sequence blocks that produce a signal evolution. Similarly, a beaker of fat, a bone, a prosthetic hip, or other things that resonate may be analyzed, and signal evolutions retrieved from these items in response to applying selected combinations of varied sequence blocks over time under selected combinations of varied conditions. These signals may be used as baseline signatures for other objects that are analyzed.

In another embodiment, baseline signatures can be acquired from the object being analyzed. Volumes in the object may be imaged using a conventional technique and may also be subjected to NMR fingerprinting. For example, 1% of a leg may be imaged conventionally and also processed using example NMR fingerprinting to establish baseline signatures for bone and other tissues. The 1% may be processed to calibrate an apparatus or method. With the calibration and baseline signatures acquired, the remaining 99% may be analyzed using NMR fingerprinting that relies on the baseline signatures established by processing the 1%. Even if some volumes produce a signal for which no fingerprinting match can be made, those volumes may simply be analyzed using a conventional approach. Thus, in one embodiment, a combination conventional and fingerprinting approach may be used to establish signatures and for calibration.

Using pattern matching to compare acquired signal evolutions to known signal evolutions may include analyzing a cross-correlation between signal evolutions of different tissues acquired using sequence blocks having different parameters. Ideally, a signal evolution would fit to exactly one member of the multi-dimensional set of known evolutions. However, a signal evolution may have relationships with more than one reference signal. Thus, in one embodiment, a result of comparing a signal evolution to a reference signal may be an identification of a reference signal with which the signal evolution is related and a measurement characterizing the relationship. For example, a signal evolution may be identified as matching a reference signal to within a desired tolerance. Similarly, a signal evolution may be identified as being x% likely to match a reference signal. In another embodiment, a signal evolution may be identified as being a weighted sum of a number of reference signals. One dimension of the multi-dimensional set could, for example, be associated with a first set of acquisition and/or excitation parameters while a second dimension of the multi-dimensional set could, for example, be associated with a second set of excitation and/or acquisition parameters. Over time, the members of the multi-dimensional set could be adapted based on fits that are achieved from live data. Over time, sequence blocks and/or combinations of sequence blocks that yield a more identity-matrix like result may be favored over sequence blocks that yield a matrix with more off-diagonal contributions. This adaptation of sequence blocks and/or series of sequence blocks based on observed results may contribute, for example, to calibrating a particular NMR apparatus for MR fingerprinting.

The following includes definitions of selected terms employed herein. The definitions include various examples and/or forms of components that fall within the scope of a term and that may be used for implementation. The examples are not intended to be limiting. Both singular and plural forms of terms may be within the definitions.

References to "one embodiment", "an embodiment", "one example", "an example", and so on, indicate that the embodiment(s) or example(s) so described may include a particular feature, structure, characteristic, property, element, or limitation, but that not every embodiment or example necessarily includes that particular feature, structure, characteristic, property, element or limitation. Furthermore, repeated use of the phrase "in one embodiment" does not necessarily refer to the same embodiment, though it may.

"Computer-readable medium", as used herein, refers to a non-transitory medium that stores signals, instructions and/or data. A computer-readable medium may take forms, including, but not limited to, non-volatile media, and volatile media. Non-volatile media may include, for example, optical disks, magnetic disks, and so on. Volatile media may include, for example, semiconductor memories, dynamic memory, and so on. Common forms of a computer-readable medium may include, but are not limited to, a floppy disk, a flexible disk, a hard disk, a magnetic tape, other magnetic medium, an ASIC, a CD, other optical medium, a RAM, a ROM, a memory chip or card, a memory stick, and other media from which a computer, a processor or other electronic device can read.

"Logic", as used herein, includes but is not limited to hardware, firmware, software in execution on a machine, and/or combinations of each to perform a function(s) or an action(s), and/or to cause a function or action from another logic, method, and/or system. Logic may include a software controlled microprocessor, a discrete logic (e.g., ASIC), an analog circuit, a digital circuit, a programmed logic device, a memory device containing instructions, and so on. Logic may include one or more gates, combinations of gates, or other circuit components. Where multiple logical logics are described, it may be possible to incorporate the multiple logical logics into one physical logic. Similarly, where a single logical logic is described, it may be possible to distribute that single logical logic between multiple physical logics.

An "operable connection", or a connection by which entities are "operably connected", is one in which signals, physical communications, and/or logical communications may be sent and/or received. An operable connection may include a physical interface, an electrical interface, and/or a data interface. An operable connection may include differing combinations of interfaces and/or connections sufficient to allow operable control. For example, two entities can be operably connected to communicate signals to each other directly or through one or more intermediate entities (e.g., processor, operating system, logic, software). Logical and/or physical communication channels can be used to create an operable connection.

"Signal", as used herein, includes but is not limited to, electrical signals, optical signals, analog signals, digital signals, data, computer instructions, processor instructions, messages, a bit, a bit stream, or other means that can be received, transmitted and/or detected.

"User", as used herein, includes but is not limited to one or more persons, software, computers or other devices, or combinations of these.

Some portions of the detailed descriptions that follow are presented in terms of algorithms and symbolic representations of operations on data bits within a memory. These algorithmic descriptions and representations are used by those skilled in the art to convey the substance of their work to others. An algorithm, here and generally, is conceived to be a sequence of operations that produce a result. The operations may include physical manipulations of physical quantities. Usually, though not necessarily, the physical quantities take the form of electrical or magnetic signals capable of being stored, transferred, combined, compared, and otherwise manipulated in a logic, and so on. The physical manipulations create a concrete, tangible, useful, real-world result.

It has proven convenient at times, principally for reasons of common usage, to refer to these signals as bits, values, elements, symbols, characters, terms, numbers, and so on. It should be borne in mind, however, that these and similar terms are to be associated with the appropriate physical quantities and are merely convenient labels applied to these quantities. Unless specifically stated otherwise, it is appreciated that throughout the description, terms including processing, computing, determining, and so on, refer to actions and processes of a computer system, logic, processor, or similar electronic device that manipulates and transforms data represented as physical (electronic) quantities.

Example methods may be better appreciated with reference to flow diagrams. While for purposes of simplicity of explanation, the illustrated methodologies are shown and described as a series of blocks, it is to be appreciated that the methodologies are not limited by the order of the blocks, as some blocks can occur in different orders and/or concurrently with other blocks from that shown and described. Moreover, less than all the illustrated blocks may be required to implement an example methodology. Blocks may be combined or separated into multiple components. Furthermore, additional and/or alternative methodologies can employ additional, not illustrated blocks.

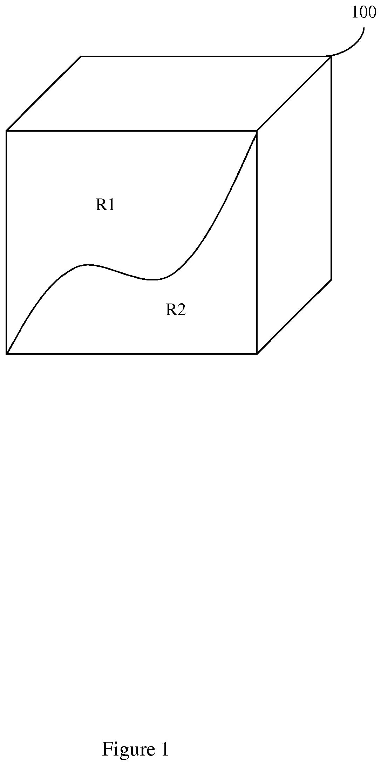

Figure 1 illustrates a volume 100 (e.g., voxel) that contains two resonant species R1 and R2. R1 and R2 may have different properties (e.g., relaxation parameters, non-relaxation parameters). For example, the T1R1 may be less than T1R2 while T2R1 may be greater than T2R2. In another example, a spin density for R1 may differ from a spin density for R2. Conventional systems may acquire a T1 weighted data set and then acquire a T2 weighted data set and then register the data sets. Example apparatus and methods apply RF energy in a series of varied sequence blocks that cause volume 100 to simultaneously produce different NMR signals from both R1 and R2. A signal evolution can be produced from these simultaneously produced different NMR signals. Information including relaxation parameters (e.g., Tl, T2), and non-relaxation parameters (e.g., diffusion coefficient, spin density, proton density, magnetic field strength) is determined from the signal evolution by comparing the acquired signal to reference information. In one embodiment, the comparing may include pattern matching to other signal evolutions for which relaxation parameters are known. The resonant species R1 and R2 can then be characterized. Since different tissues have different known properties (e.g., relaxation parameters, non-relaxation parameters), different tissues can be identified using the characterization. While two resonant species are illustrated, one skilled in the art will appreciate that a volume may include a greater or lesser number of resonant species. Therefore, example methods and apparatus apply more generally to a volume having multiple resonant species.

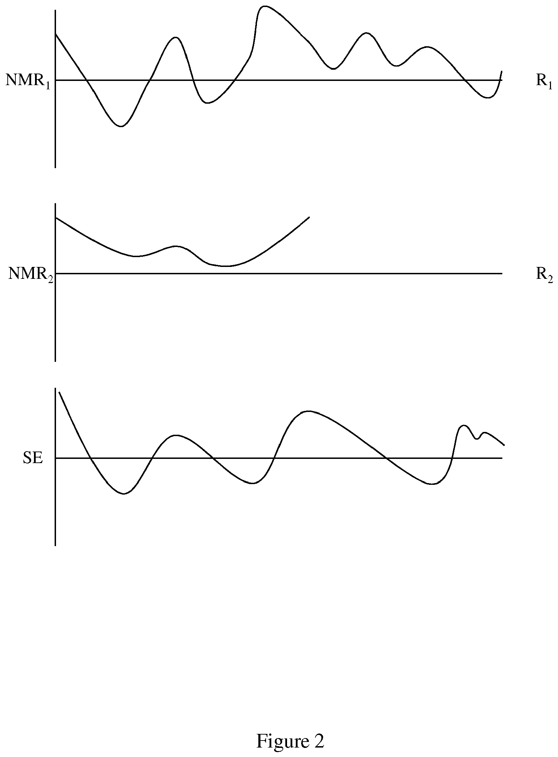

Figure 2 illustrates plots of two individual NMR signals NMR1 and NMR2 received from the two resonant species R1 and R2 in volume 100. NMR1 includes data points generated by R1 under different conditions at different times. NMR2 includes data points generated by R2 under the different conditions at the different times. Signal evolution SE results from NMR1 and NMR2 being generated and acquired simultaneously. The space from which the data points for NMR1 and NMR2 is acquired may be referred to as a (k, t, E) space, where in different examples, E refers to (Tl, T2, D), (Tl, T2, D, ...), (Tl, T2, ...) where D refers to diffusion relaxation. In one example, both t and E may be non-linear. In another example, both t and E may be pseudo-random. Once again, while two plots associated with two resonant species are illustrated, one skilled in the art will appreciate that a volume may include a greater or lesser number of resonant species and thus may produce a greater or lesser number of signals. Therefore, example methods and apparatus apply more generally to a volume having one or more resonant species.

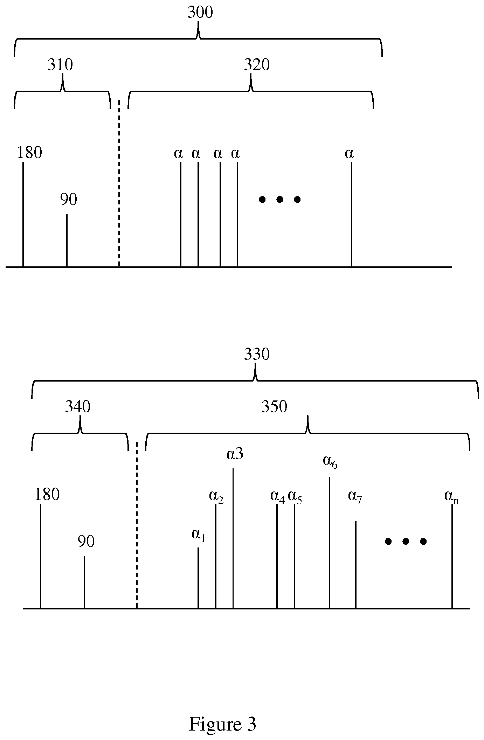

Figure 3 compares and contrasts conventional sequence blocks to example sequence blocks. Sequence block 300 includes a preparation phase 310 and an acquisition phase 320. During acquisition phase 320, multiple acquisitions using the same flip angle and the same interval between acquisitions may be performed. Acquisition phase 320 resembles the Doneva approach, which acquires data from a (k, t) space, where t varies either constantly or linearly. The constant variation facilitates acquiring signal with constant amplitude and phase as required for conventional image reconstruction.

Sequence block 330 also includes a phase 340 and an acquisition phase 350. Notice that acquisition phase 350 is much longer than acquisition phase 320. Unlike acquisition phase 320 where parameters are either fixed or vary linearly, in acquisition phase 350 the parameters vary widely, either non-linearly, randomly, and/or pseudo-randomly. Parameters that may vary include, but are not limited to, echo time, flip angle, phase encoding, and others. Note also that while phase 340 may, in some examples, be a preparation phase or preparation-like phase, that phase 340 does not necessarily perform a conventional preparation.

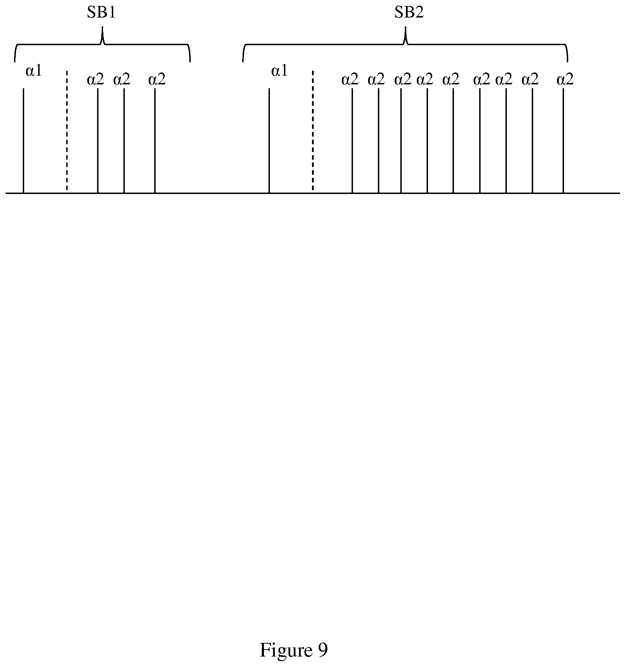

Figure 9 illustrates another example set of sequence blocks. In figure 9, a first sequence block SB1 has a first alpha pulse al and a series of identical a2 pulses. In figure 9, a second sequence block SB2 has the same first alpha pulse al and a different series of identical a2 pulses. The phase may be the same for the a2 pulses. Thus, in this example, the only difference between members of the set of sequence blocks is the number of a2 pulses. One skilled in the art will appreciate that other sets of sequence blocks may be employed.

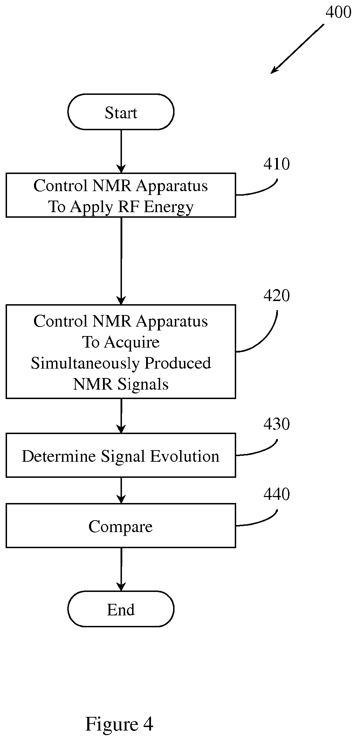

Figure 4 illustrates a method 400 associated with NMR fingerprinting. Method 400 includes, at 410, controlling an NMR apparatus to apply RF energy to a volume in an object. The volume may contain one or more resonant species. In one embodiment, the object may be a human and thus resonant species may include, but are not limited to, tissue, fat, water, hydrogen, and prosthetics.

The RF energy is be applied in a series of variable sequence blocks. Sequence blocks may vary in a number of parameters including, but not limited to, echo time, flip angle, phase encoding, diffusion encoding, flow encoding, RF pulse amplitude, RF pulse phase, number of RF pulses, type of gradient applied between an excitation portion of a sequence block and a readout portion of a sequence block, number of gradients applied between an excitation portion of a sequence block and a readout portion of a sequence block, type of gradient applied between a readout portion of a sequence block and an excitation portion of a sequence block, number of gradients applied between a readout portion of a sequence block and an excitation portion of a sequence block, type of gradient applied during a readout portion of a sequence block, number of gradients applied during a readout portion of a sequence block, amount of RF spoiling, and amount of gradient spoiling. In different embodiments two, three, four, or more parameters may vary between sequence blocks. According to the invention, the number of parameters varied between sequence blocks itself is varied at least once during the series of variable sequence blocks. For example, AI (sequence block 1) may differ from A2 in five parameters, A2 may differ from A3 in seven parameters, and A3 may differ from A4 in two parameters. One skilled in the art will appreciate that there are a nearly infinite number of series of sequence blocks that can be created by varying this large number of parameters. In one embodiment, a series of sequence blocks is crafted so that the series have different amounts (e.g., 1%, 2%, 5%, 10%, 50%, 99%, 100%) of unique sequence blocks as defined by their varied parameters. In different embodiments, a series of sequence blocks may include more than ten, more than one hundred, more than one thousand, more than ten thousand, and more than one hundred thousand sequence blocks. In one example, the only difference between consecutive sequence blocks may be the number of $\alpha2$ pulses as illustrated in figure 9.

The RF energy applied during a sequence block is configured to cause different individual resonant species to simultaneously produce individual NMR signals. Unlike conventional systems, according to the invention, N of the sequence block parameters are varied either non-linearly, randomly, and/or pseudo-randomly during the series of variable sequence blocks, N being an integer greater than one, and wherein N itself is varied at least once during the series of variable sequence blocks. One skilled in the art will grasp that the signal content of a signal evolution may vary directly with N. Thus, as more parameters are varied, a potentially richer signal is retrieved. Conventionally, a signal that depends on a single parameter is desired and required to facilitate imaging. Here, acquiring signals with greater information content facilitates producing more distinct and identifiable signal evolutions.

In one embodiment, the NMR apparatus may be controlled at 410 to apply members of the series of variable sequence blocks according to a partially random acquisition plan configured to under-sample the object at an under-sampling rate R. In different embodiments, rate R may be, for example, two, four, or greater.

Method 400 also includes, at 420, controlling the NMR apparatus to acquire the simultaneously produced individual NMR signals. Unlike conventional systems where the time during which an NMR signal can be acquired is severely limited (e.g., 4-5 seconds), the NMR apparatus can be controlled to acquire NMR signal for significantly longer periods of time. For example, the NMR apparatus can be controlled to acquire signal for up to ten seconds, for up to twenty seconds, for up to one hundred seconds, or longer. NMR signals can be acquired for longer periods of time because signal information content remains viable for longer periods of time in response to the series of varied RF energy applied at 410. In different embodiments, the information content in the signal evolution may remain above an information content threshold for at least five seconds, for at least ten seconds, for at least sixty seconds, or for longer. An information content threshold may describe, for example, the degree to which a subsequent signal acquisition includes information that can be retrieved and that differs from information acquired in a previous signal acquisition. For example, a signal that has no retrievable information would likely fall below an information content threshold while a signal with retrievable information that differs from information retrieved from a previous signal would likely be above the information content threshold.

Method 400 also includes, at 430, controlling the NMR apparatus to determine a signal evolution from the acquired NMR signals. Determining the signal evolution may include storing (k, t, E) space data points acquired during action 420. While an individual sequence block may yield a single point in (k, t, E) space, the signal evolution is determined by the series of variable sequence blocks. Overtime, series of variable sequence blocks that yield particularly useful signal evolutions may be identified.

In one embodiment, the simultaneously produced signals are acquired at 420 over a first period of time and the signal evolution is determined at 430 over a second period of time. In different embodiments the first period of time may be ten seconds or longer, sixty seconds or longer, and even longer. Additionally, in different embodiments, the second period of time may be ten seconds or longer, sixty seconds or longer, and even longer.

Method 400 also includes, at 440, controlling the NMR apparatus to compare first information to reference information. The first information is the signal evolution. The reference information may be, for example, known, stored, simulated, and/or predicted signal evolutions. The reference information may also include information that is produced as a function of a known, stored, simulated, or predicted signal evolution. The reference information may be produced by, for example, transforming a signal evolution, combing signal evolutions, decomposing signal evolutions, and other operations. In different examples, the "stored" signal evolutions may include previously acquired signals, simulated signals, or both. In one embodiment, the stored signal evolutions are associated with signals not acquired from the object while in another embodiment the stored signal evolutions are associated with signals acquired from the object. In one embodiment, the stored signals may be associated with signals acquired from the object being analyzed and signals not acquired from the object being analyzed.

The stored signals and information derived from reference signal evolutions may be associated with a potentially very large data space. Thus, one skilled in the art will appreciate that the stored signal evolutions and information derived from reference signal evolutions may include signals outside the set of signal evolutions characterized by:

$$SE = A - Be_{-t/c}$$

where:

SE is a signal evolution,
A is a constant,
B is a constant,
t is time, and
C is a single relaxation parameter.

Indeed, one skilled in the art will appreciate that the very large data space for signal evolutions can be partially described by:

$$SE = \prod_{i=1}^{N_A} \sum_{j=1}^{N_{RF}} R_i(\alpha) R_{RF_{ij}}(\alpha, \varphi) R(G) E_i(T1, T2, D)$$

where:

SE is a signal evolution,
$N_A$ is a number of sequence blocks,
$N_{RF}$ is a number of RF pulses in a sequence block,
$\alpha$ is a flip angle,
$\Phi$ is a phase angle,
$Ri(\alpha)$ is a rotation due to off resonance,
$R_{RFij}(\alpha, \Phi)$ is a rotation due to RF differences,
$R(G)$ is a rotation due to a gradient,
T1 is spin-lattice relaxation,
T2 is spin-spin relaxation,
D is diffusion relaxation, and

$E_i(T1,T2,D)$ is associated with magnetization changes.

While $E_i(T1,T2,D)$ is provided as an example, one skilled in the art will appreciate that in different embodiments, $E_i(T1,T2,D)$ may actually be $E_i(T1,T2,D,...)$, or $E_i(T1,T2,...)$.

In one example, the summation on j could be replaced by a product on j, e.g.:

$$SE = \prod_{i=1}^{N_A} \prod_{j=1}^{N_{RF}} R_i(\alpha) R_{RF_{ij}}(\alpha, \varphi) R(G) E_i(T1, T2, D)$$

In NMR, MRI, or ESR (electron spin resonance), a Bloch equation is a member of a set of macroscopic equations that are used to calculate the nuclear magnetization $M = (M_x, M_y, M_z)$ as a function of time when relaxation times $T_1$ and $T_2$ are present. These phenomenological equations were introduced by Felix Bloch and may also be referred to as the equations of motion of nuclear magnetization. One skilled in the art will appreciate that in one embodiment $Ri(\alpha)$, $R_{RF_{ij}}(\alpha,\Phi)$, and $R(G)$ may be viewed as Bloch equations.

While Figure 4 illustrates various actions occurring in serial, it is to be appreciated that various actions illustrated in Figure 4 could occur substantially in parallel. By way of illustration, a first process could control applying RF energy, a second process could control acquiring NMR signals and determining a signal evolution, and a third process could perform comparisons. While three processes are described, it is to be appreciated that a greater and/or lesser number of processes could be employed.

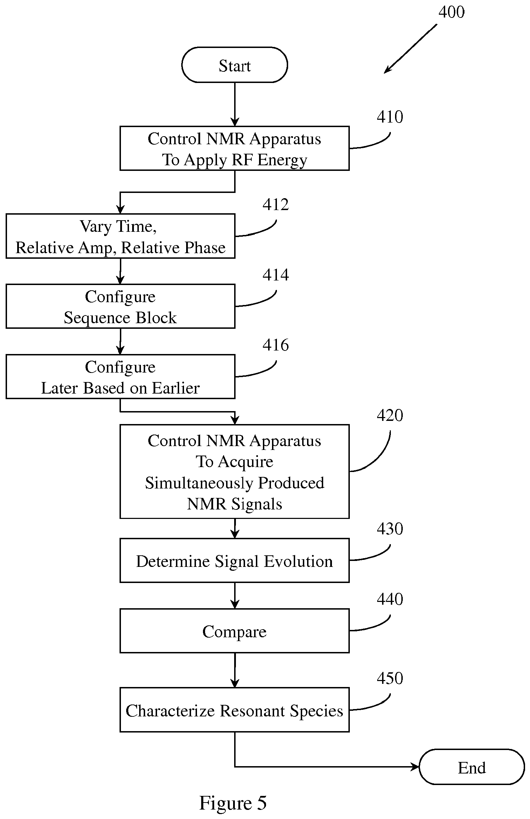

Figure 5 illustrates another embodiment of method 400 (Figure 4). This embodiment includes actions 410, 420, 430, and 440. However, this embodiment also includes actions 412, 414, 416, and 450.

This embodiment of method 400 includes, at 412, controlling the NMR apparatus to vary one or more of, the amount of time between sequence blocks, the relative amplitude of sequence blocks, and the relative phase of sequence blocks. Thus, not only can the individual parameters (e.g., flip angle, phase) be varied between sequence blocks, but the times between sequence blocks and other differences between sequence blocks can be varied. This facilitates creating additional signal content in the signal evolution.

This embodiment of method 400 also includes, at 414, controlling the NMR apparatus to configure a member of the series of variable sequence blocks as one of, a TrueFISP pulse sequence, a FLASH pulse sequence, and a TSE pulse sequence. Action 414 illustrates that a set of sequence blocks is not necessarily the same thing as a conventional pulse sequence. A sequence block differs from a conventional pulse sequence for at least the reason that non-linearly varying $\Delta t$ and $\Delta E$, which produce NMR signals in (k, t, E) space having non-constant amplitudes and phases are encouraged, not prohibited.

This embodiment of method 400 also includes, at 416, controlling the NMR apparatus to configure a later member of the series of variable sequence blocks based, at least in part, on an NMR signal acquired in response to applying an earlier member of the series of variable sequence blocks. Thus, this embodiment of method 400 is an adaptive method where the order of members of the series of varied sequence blocks may not be known ahead of time. Instead, as data points in (k, t, E) space are acquired, and as a signal evolves, decisions concerning different sequence blocks and different sets of parameters to vary may be made. By way of illustration, a first number of data points in (k, t, E) space and an evolving signal may be leading towards one relaxation parameter determination and away from another relaxation parameter determination. Therefore, sequence blocks that can confirm and/or reject either of these leads may be applied next in the series to facilitate a guided and more rapid convergence in the pattern matching process.

This embodiment of method 400 also includes, at 450, controlling the NMR apparatus to characterize at least one of the resonant species. In one embodiment, the characterizing may be a function of comparing the signal evolution to one or more stored (e.g., known, simulated, predicted) signal evolutions. Comparing the acquired signal evolution to a stored signal evolution may include, for example, controlling the NMR apparatus to compare the signal evolution to members of a multi-dimensional set of NMR signal evolutions. A first dimension in the multi-dimensional set may be associated with a first set of sequence block parameters and a second dimension in the multi-dimensional set may be associated with a second, different set of sequence block parameters. Since a signal evolution evolves over time, the multi-dimensional set may include a time dimension and the pattern matching process may include a path matching process that monitors the progress of the signal evolution. Additionally, since one series of varied sequence blocks may differ from another series of varied sequence blocks, the multi-dimensional set may include an order dimension where once again the pattern matching process may path match as opposed to just pattern matching.

Characterizing a resonant species may include, for example, identifying relaxation parameters including, but not limited to, T1 relaxation associated with the resonant species, T2 relaxation associated with the resonant species, off-resonance relaxation associated with the resonant species, and diffusion weighted relaxation associated with the resonant species. Characterizing a resonant species may also include, for example, identifying properties that are not relaxation parameters including, but not limited to, diffusion coefficients, spin density, proton density, magnetic field strength, gradient field strength, tissue type, and material type.

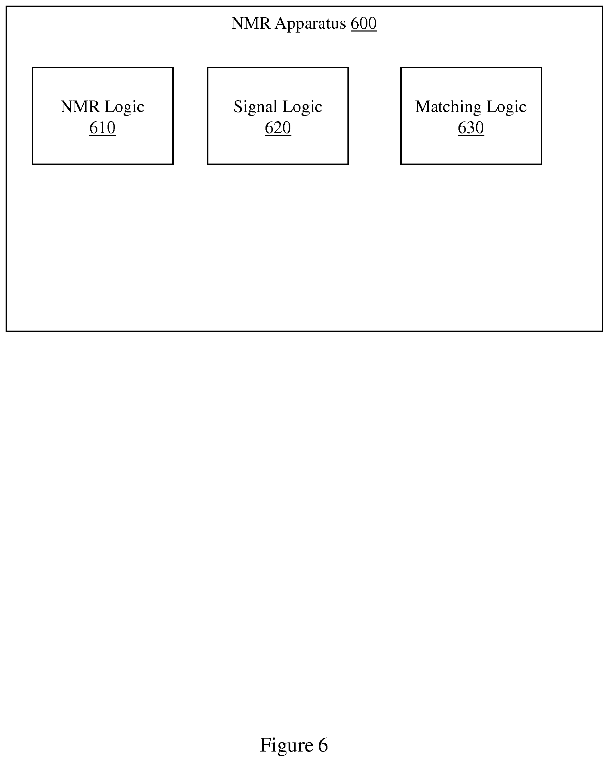

Figure 6 illustrates an NMR apparatus 600. NMR apparatus 600 includes an NMR logic 610. NMR logic 610 is configured to repetitively and variably sample an object in a (k, t, E) space to acquire a set of NMR signals that may have non-constant amplitude and/or phase. Members of the set of NMR signals are associated with different points in the (k, t, E) space. In different embodiments the different points are sampled according to a plan where t and/or E varies non-linearly and/or in a non-constant manner.

NMR apparatus 600 also includes a signal logic 620. Signal logic 620 is configured to produce an NMR signal evolution from the NMR signals. The signal evolution may include a number of NMR signals acquired over a period of time.

NMR apparatus 600 also includes a matching logic 630. Matching logic 630 is configured to compare the produced NMR signal evolution or information associated with the produced NMR signal evolution to reference information. The reference information may be, for example, a previously acquired signal evolution, a simulated signal evolution, an item derived from a signal evolution other than the produced NMR signal evolution, and other information.

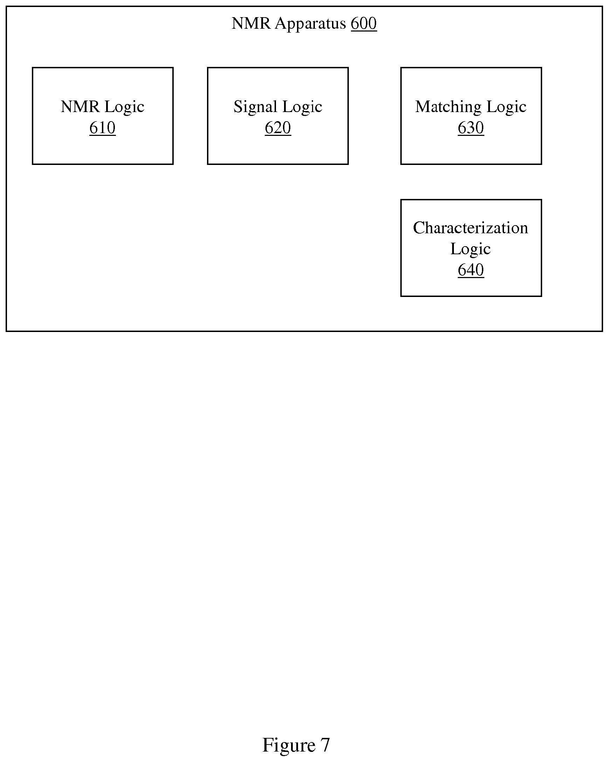

Figure 7 illustrates another embodiment of apparatus 600 (Figure 6). This embodiment of apparatus 600 includes a characterization logic 640. Characterization logic 640 is configured to characterize a resonant species in the object. Characterizing the resonant species may include comparing the NMR signal evolution or information derived from the NMR signal evolution to reference information. The reference information may include, for example, a characterizing signal evolution(s), information derived from a characterizing signal evolution(s), and other information. Characterizing the resonant species may include identifying relaxation parameters including, but not limited to, T1 relaxation, T2 relaxation, diffusion weighted relaxation, and off-resonance relaxation. Characterizing the resonant species may also include identifying non-relaxation parameters including, but not limited to, diffusion co-efficient, spin density, proton density, tissue type, and material type.

While matching logic 630 (Figure 6) and characterization logic 640 (Figure 7) are illustrated as being part of NMR apparatus 600, in one embodiment, the matching logic 630 and/or the characterization logic 640 may reside in an apparatus separate from the NMR apparatus 600. In this embodiment, NMR apparatus 600 may provide NMR signals to the separate apparatus housing matching logic 630 and characterization logic 640. In one embodiment, matching logic 630 and characterization logic 640 may reside in separate apparatus.

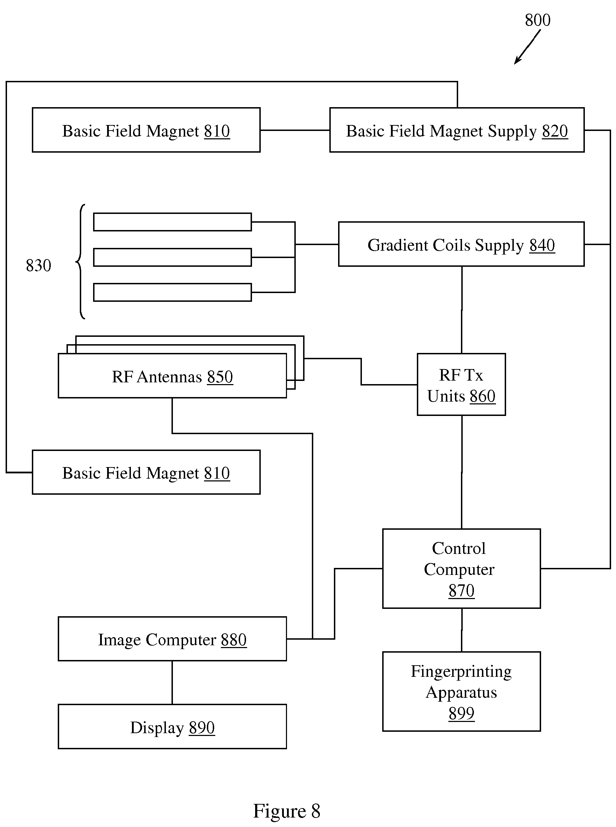

Figure 8 illustrates an example MR apparatus 800 configured with a fingerprinting apparatus 899 to facilitate MR fingerprinting. The fingerprinting apparatus 899 may be configured with elements of example apparatus described herein and/or may perform example methods described herein. While fingerprinting apparatus 899 is illustrated as part of MR apparatus 800, in one example, fingerprinting apparatus 899 may be a separate apparatus or apparatuses.

The apparatus 800 includes a basic field magnet(s) 810 and a basic field magnet supply 820. Ideally, the basic field magnets 810 would produce a uniform B0 field. However, in practice, the B0 field may not be uniform, and may vary over an object being analyzed by the MR apparatus 800. MR apparatus 800 may include gradient coils 830 configured to emit gradient magnetic fields like GS, GP and GR. The gradient coils 830 may be controlled, at least in part, by a gradient coils supply 840. In some examples, the timing, strength, and orientation of the gradient magnetic fields may be controlled, and thus selectively adapted, during an MR procedure.

MR apparatus 800 may include a set of RF antennas 850 that are configured to generate RF pulses and to receive resulting nuclear magnetic resonance signals from an object to which the RF pulses are directed. In some examples, how the pulses are generated and how the resulting MR signals are received may be controlled and thus may be selectively adapted during an MR procedure. Separate RF transmission and reception coils can be employed. The RF antennas 850 may be controlled, at least in part, by a set of RF transmission units 860. An RF transmission unit 860 may provide a signal to an RF antenna 850.

The gradient coils supply 840 and the RF transmission units 860 may be controlled, at least in part, by a control computer 870. In one example, the control computer 870 may be programmed to control an NMR device as described herein. Conventionally, the magnetic resonance signals received from the RF antennas 850 can be employed to generate an image and thus may be subject to a transformation process like a two dimensional FFT that generates pixilated image data. The transformation can be performed by an image computer 880 or other similar processing device. The image data may then be shown on a display 890.

However, fingerprinting apparatus 899 facilitates not having to do conventional reconstruction of an image from MR signals received from the RF antennas 850. Thus the RF energy applied to an object by apparatus 800 need not be constrained to produce signals with substantially constant amplitudes or phases. Instead, fingerprinting apparatus 899 facilitates matching received signals to known signals for which a reconstruction, relaxation parameter, or other information is already available. This facilitates producing a quantitative result.

While Figure 8 illustrates an example MR apparatus 800 that includes various components connected in various ways, it is to be appreciated that other MR apparatus may include other components connected in other ways.

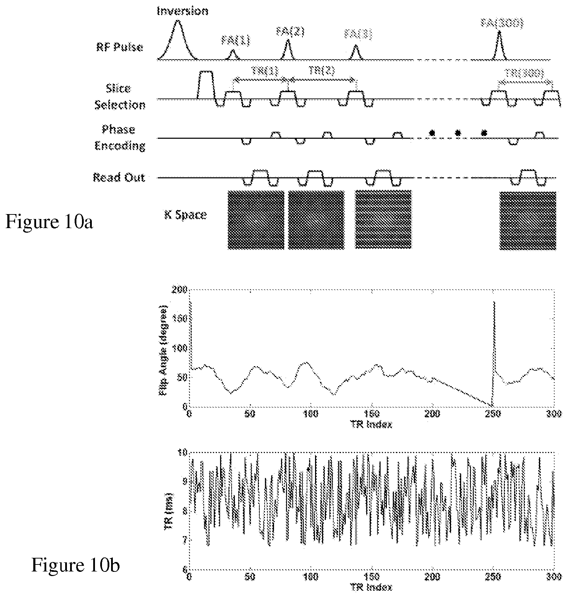

Figures 10a-10b illustrate an example magnetic resonance fingerprinting (MRF) sequence pattern. The terms nuclear magnetic resonance fingerprinting and magnetic resonance fingerprinting are used interchangeably herein. Figure 10a illustrates an example acquisition sequence diagram where in different TR various sequence components are varied in a pseudorandom pattern. This basic acquisition scheme is illustrated being repeated with different spatial encoding gradients to fully encode an image for the 300 TRs of the complete acquisition. Figure 10b illustrates an example of FA and TR patterns.

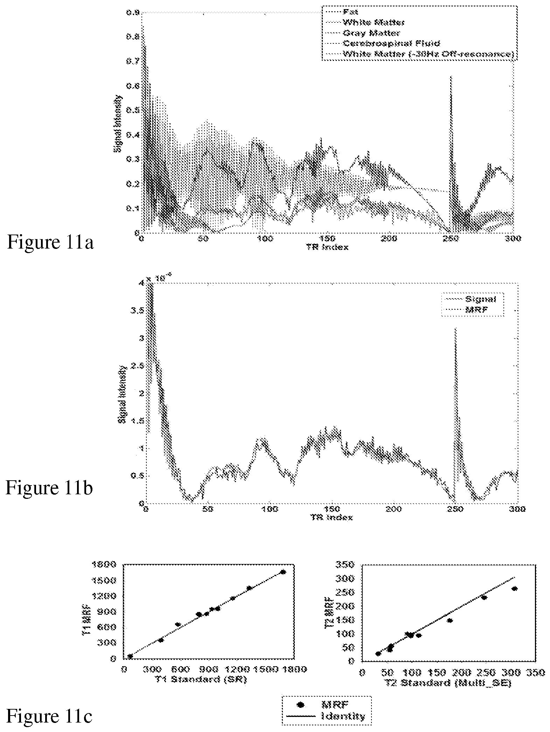

Figures 11a-11c illustrate example signal properties and matching results. Figure 11a illustrates example simulated signal evolution curves corresponding to four normal brain tissues. An example signal evolution curve from white matter with off-resonance is also plotted. Figure 11b illustrates one example of acquired signal evolution curves and a comparison to a dictionary. The retrieved T1, T2, proton density and off-resonance values are 800 ms, 60 ms, 0.85e-5, and -4 Hz respectively. Figure 11c illustrates T1 and T2 values retrieved from a matching algorithm. A comparison of signal from ten phantoms is illustrated with the values acquired from standard spin-echo sequences. The R2 values for T1 and T2 comparisons are 0.9952 and 0.986 respectively.

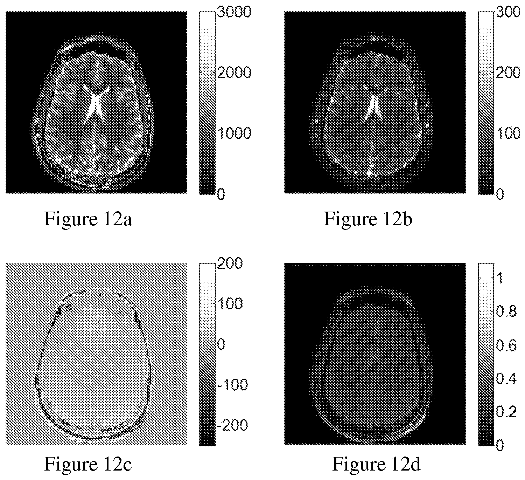

Figures 12a-12d illustrate in vivo results associated with NMR fingerprinting. Figure 12a illustrates a T1 map (ms), Figure 12b illustrates a T2 map (ms), Figure 12c illustrates an off-resonance map (Hz), and Figure 12d illustrates a proton density map. Information for producing figures 12a-12d was acquired simultaneously using one example MRF.

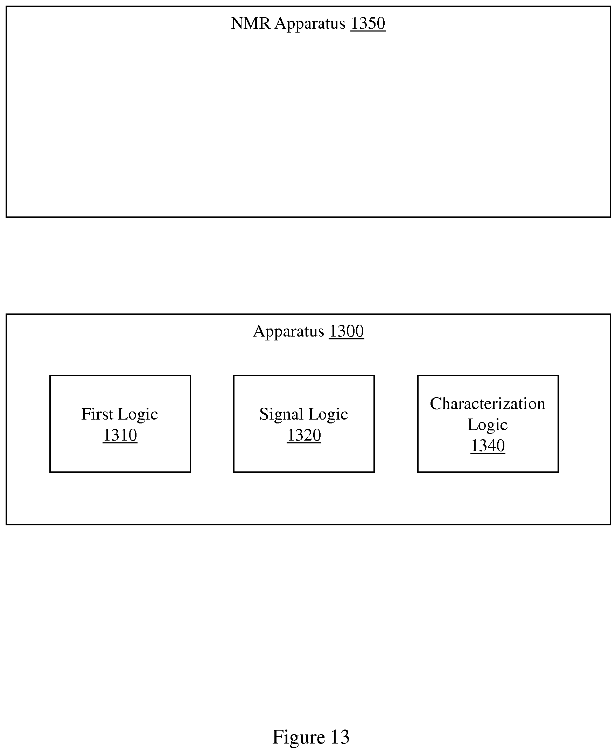

Figure 13 illustrates an apparatus 1300 configured to compare acquired information to reference information. Apparatus 1300 includes a first logic 1310 configured to receive a first set of data from an NMR apparatus 1350. NMR apparatus 1350 is configured to repetitively and variably sample a (k, t, E) space associated with an object to acquire a set of NMR signals. Members of the set of data are associated with different points in the (k, t, E) space, where t is time and E includes at least one of, T1, T2, and one other parameter, T1 being spin-lattice relaxation, and T2 being spin-spin relaxation, and where one or more of, t, and E, vary non-linearly.

Apparatus 1300 also includes a signal logic 1320 that is configured to produce an NMR signal evolution from the first set of data. In one embodiment, the (k, t, E) space is a produced as a function of applying RF energy to an object according to two or more different sequence blocks. Recall that a sequence block includes one or more excitation phases, one or more readout phases, and one or more waiting phases, and that at least one member of the two or more sequence blocks differs from at least one other member of the two or more sequence blocks in at least one of, the number of $\alpha2$ pulses in a sequence block, the spacing of $\alpha2$ pulses in a sequence block, the phase of $\alpha2$ pulses in a sequence block, and the amplitude of $\alpha2$ pulses in a sequence block.

Apparatus 1300 also includes a characterization logic 1340 that is configured to characterize the object based, at least in part, on comparing the first set of data to a reference set of data. In one embodiment, the characterization logic 1340 may be configured to provide image pixel data suitable for producing a diagnostic image. The image pixel data is identified from comparisons between the first set of data and the reference set of data and between the reference set of data and the image pixel data. In another embodiment, the characterization logic 1340 may be configured to provide diagnostic information. The diagnostic information is identified from comparisons between the first set of data and the reference set of data and between the reference set of data and the image pixel data.

In one embodiment, characterizing the object includes providing information concerning items including, but not limited to, T1 associated with the object, T2 associated with the object, a diffusion coefficient associated with the object, a spin density associated with the object, a proton density associated with the object, a magnetic field to which the object was exposed, a gradient field to which the object was exposed, a tissue type of the object, and an identification of the object.

In one embodiment, characterizing the object may include performing actions including, but not limited to, identifying a portion of the reference set of data related to the first set of data, identifying a degree to which a portion of the reference set of data is related to the first set of data, and identifying the likelihood that a portion of the reference set of data is related to the first set of data.

In one embodiment, the first set of data may have data including, but not limited to, the NMR signals acquired from the object in response to the NMR fingerprinting excitation, and a signal evolution produced from the NMR signals acquired from the object in response to the NMR fingerprinting excitation.

In different embodiments, the reference set of data may include, but is not limited to including, a previously acquired NMR signal, a modeled NMR signal, a previously acquired signal evolution, and a modeled signal evolution.

In one embodiment, comparing the first set of data to the reference set of data may include, but is not limited to, pattern matching, selecting, minimizing, and optimizing. Pattern matching may include, but is not limited to, orthogonal matching pursuit, categorical sequence labeling, regression, clustering, classification, real valued sequence labeling, parsing, Bayesian methods, Markov methods, ensemble learning methods, and template matching. Optimization may include, but is not limited to, least squares optimization, regularized least squares optimization, basis pursuit optimization, and matching pursuit optimization.

While example systems, methods, and so on have been illustrated by describing examples, and while the examples have been described in considerable detail, it is not the intention of the applicants to restrict or in any way limit the scope of the appended claims to such detail. It is, of course, not possible to describe every conceivable combination of components or methodologies for purposes of describing the systems, methods, and so on described herein. Therefore, the invention is not limited to the specific details, the representative apparatus, and illustrative examples shown and described. Thus, this application is intended to embrace alterations, modifications, and variations that fall within the scope of the appended claims.

To the extent that the term "includes" or "including" is employed in the detailed description or the claims, it is intended to be inclusive in a manner similar to the term "comprising" as that term is interpreted when employed as a transitional word in a claim.

To the extent that the term "or" is employed in the detailed description or claims (e.g., A or B) it is intended to mean "A or B or both". When the applicants intend to indicate "only A or B but not both" then the term "only A or B but not both" will be employed. Thus, use of the term "or" herein is the inclusive, and not the exclusive use. See, Bryan A. Garner, A Dictionary of Modern Legal Usage 624 (2d. Ed. 1995).

To the extent that the phrase "one of, A, B, and C" is employed herein, (e.g., a data store configured to store one of, A, B, and C) it is intended to convey the set of possibilities A, B, and C, (e.g., the data store may store only A, only B, or only C). It is not intended to require one of A, one of B, and one of C. When the applicants intend to indicate "at least one of A, at least one of B, and at least one of C", then the phrasing "at least one of A, at least one of B, and at least one of C" will be employed.

To the extent that the phrase "one or more of, A, B, and C" is employed herein, (e.g., a data store configured to store one or more of, A, B, and C) it is intended to convey the set of possibilities A, B, C, AB, AC, BC, ABC, AA...A, BB...B, CC...C, AA...ABB...B, AA...ACC...C, BB...BCC...C, or AA...ABB...BCC...C (e.g., the data store may store only A, only B, only C, A&B, A&C, B&C, A&B&C, or other combinations thereof including multiple instances of A, B, or C). It is not intended to require one of A, one of B, and one of C. When the applicants intend to indicate "at least one of A, at least one of B, and at least one of C", then the phrasing "at least one of A, at least one of B, and at least one of C" will be employed.

**Claims**

1. A method (400), comprising:
   controlling a nuclear magnetic resonance (NMR) apparatus (600) to expose a material to an NMR fingerprinting excitation by:

   controlling the NMR apparatus
   to apply (410) radio frequency (RF) energy to the material in a series of variable sequence blocks (330), wherein a sequence block (330) has a plurality of sequence block parameters and includes one or more excitation phases, one or more readout phases, and one or more waiting phases, wherein N of said sequence block parameters are varied either non-linearly, randomly, and/or pseudo-randomly during the series of variable sequence blocks (330), N being an integer greater than one,

   wherein N itself is varied at least once during the series of variable sequence blocks (330),
   wherein the material contains one or more resonant species (R1; R2),
   wherein the RF energy applied during a sequence block (330) is configured to cause the one or more resonant species (R1; R2) in the material to simultaneously produce individual NMR signals (NMR1; NMR2), and

   controlling the NMR apparatus to acquire (420) the produced NMR signals and to determine (430) an NMR signal evolution (SE) from the acquired NMR signals (NMR1; NMR2),
   wherein the signal evolution (SE) is determined by the series of variable sequence blocks (330);
   determining one or more parameters of the material for characterizing (450) a property of the material by comparing (440) the determined NMR signal evolution(SE) associated with NMR signals (NMR1; NMR2) acquired from the material in response to the NMR fingerprinting excitation to reference NMR signal evolutions for which said one or more parameters are known.

2. The method of claim 1, wherein the one or more parameters of the material are one or more of, relaxation parameters such as T1 associated with the material or T2 associated with the material and non-relaxation parameters such as a diffusion coefficient associated with the material, a spin density associated with the material, a proton density associated with the material, a magnetic field to which the material was exposed, a magnetic gradient field to which material was exposed, a tissue type of the material, and an identification of the material, T1 being spin-lattice relaxation, and T2 being spin-spin relaxation.

3. The method of claim 2, wherein the step of comparing includes one or more of, identifying a portion of the reference NMR evolutions related to the the determined NMR signal evolution (SE), identifying a degree to which a portion of the reference NMR evolutions is related to the the determined NMR signal evolution (SE), and identifying the likelihood that a portion of the reference NMR evolutions is related to the the determined NMR signal evolution (SE).

4. The method of claim 1, where the reference NMR signal evolution includes one or more of, a previously acquired signal evolution, a modeled signal evolution, and a simulated signal evolution.

5. The method of claim 1, where the step of comparing includes one or more of, pattern matching, selecting, minimizing, and optimizing.

6. The method of claim 5, where pattern matching includes one or more of, orthogonal matching pursuit, categorical sequence labeling, regression, clustering, classification, real valued sequence labeling, parsing, Bayesian methods, Markov methods, ensemble learning methods, and template matching.

7. The method of claim 5, where optimization includes one or more of, least squares optimization, regularized least squares optimization, basis pursuit optimization, and matching pursuit optimization.

8. The method of claim 1, where the sequence block parameters comprise echo time (TE), flip angle (FA), phase encoding, diffusion encoding, flow encoding, RF pulse amplitude, RF pulse phase, number of RF pulses, type of magnetic gradient (GS; GR; GP) applied between an excitation portion of a sequence block (330) and a readout portion of a sequence block (330), number of magnetic gradients (GS; GR; GP) applied between an excitation portion of a sequence block (330) and a readout portion of a sequence block (330), type of magnetic gradient (GS; GR; GP) applied between a readout portion of a sequence block (330) and an excitation portion of a sequence block (330), number of magnetic gradients (GS; GR; GP) applied between a readout portion of a sequence block (330) and an excitation portion of a sequence block (330), type of magnetic gradient (GS; GR; GP) applied during a readout portion of a sequence block (330), number of magnetic gradients (GS; GR; GP) applied during a readout portion of a sequence block (330), amount of RF spoiling, and amount of magnetic gradient spoiling.

9. The method of claim 1, comprising:

   controlling the NMR apparatus to vary one or more of, the amount of time between sequence blocks (330) in the series of variable sequence blocks (330), the relative amplitude of RF pulses in sequence blocks (330) in the series of variable sequence blocks (330), and
   the relative phase of RF pulses in sequence blocks (330) in the series of variable sequence blocks (330).

10. The method of claim 1, where N is greater than two and where at least one percent of the members of the series of variable sequence blocks (330) are unique.

11. The method of claim 1, where the reference NMR evolutions includes signal evolutions (SE) outside the set of signal evolutions (SE) **characterized by**:

$$SE = A - Be^{-t/C}$$

where:

   SE is a signal evolution,
   A is a constant,
   B is a constant,
   t is time, and
   C is a single relaxation parameter.

12. The method of claim 1, where the reference NMR evolutions includes signal evolutions (SE) selected from a set of signals described by:

$$SE = \prod_{i=1}^{N_A} \sum_{j=1}^{N_{RF}} R_i(\alpha) R_{RFij}(\alpha, \varphi) R(G) E_i(T1, T2, D)$$

where:

SE is a signal evolution,
$N_A$ is a number of sequence blocks,
$N_{RF}$ is a number of RF pulses in a sequence block,
$\alpha$ is flip angle,
$\Phi$ is a phase angle,
$Ri(\alpha)$ is a rotation due to off resonance,
$R_{RFij}(\alpha, \Phi)$ is a rotation due to RF differences,
$R(G)$ is a rotation due to a magnetic gradient,
T1 is spin-lattice relaxation,
T2 is spin-spin relaxation,
D is diffusion relaxation, and
$Ei(T1, T2, D)$ is associated with magnetization differences.

13. The method of claim 1, where the reference NMR evolutions includes signal evolutions (SE) selected from a set of signals described by:

$$SE = \prod_{i=1}^{N_A} \prod_{j=1}^{N_{RF}} R_i(\alpha) R_{RFij}(\alpha, \varphi) R(G) E_i(T1, T2, \dots)$$

where:

SE is a signal evolution,
$N_A$ is a number of sequence blocks,
$N_{RF}$ is a number of RF pulses in a sequence block,
$\alpha$ is flip angle,
$\Phi$ is a phase angle,
$Ri(\alpha)$ is a rotation due to off resonance,
$R_{RFij}(\alpha, \Phi)$ is a rotation due to RF differences,
$R(G)$ is a rotation due to a magnetic gradient,
T1 is spin-lattice relaxation,
T2 is spin-spin relaxation, and
$E1(T1, T2, \dots)$ is associated with magnetization changes.

14. An NMR apparatus (600), comprising:

an NMR logic (610) configured to receive acquired NMR signals wherein the NMR logic (610) is configured to control an NMR apparatus to expose a material to an NMR fingerprinting excitation by:

- controlling the NMR apparatus (600) to apply radio frequency (RF) energy to the material in a series of variable sequence blocks (330), wherein a sequence block (330) has a plurality of sequence block parameters and includes one or more excitation phases, one or more readout phases, and one or more waiting phases, wherein N of said sequence block parameters are varied either non-linearly, randomly, and/or pseudo-randomly during the series of variable sequence blocks (330), N being an integer greater than one,

wherein N itself is varied at least once during the series of variable sequence blocks (330),
wherein the material contains one or more resonant species (R1; R2),
wherein the RF energy applied during a sequence block (330) is configured to cause the one or more resonant species (R1; R2) in the material to simultaneously produce individual NMR signals (NMR1; NMR2), and

- controlling the NMR apparatus (600) to acquire the produced NMR signals (NMR1; NMR2);

a signal logic (620) configured to determine a NMR signal evolution (SE) from the acquired NMR signals (NMR1; NMR2), wherein the signal evolution (SE) is determined by the series of variable sequence blocks (330); a characterization logic (640) configured to determine one or more parameters of the material for characterizing a property of the material by comparing the determined NMR signal evolution (SE) associated with NMR signals (NMR1; NMR2) acquired from the material in response to the NMR fingerprinting excitation to reference NMR signal evolutions for which said one or more parameters are known.

15. The apparatus of claim 14, where the characterization logic (640) is configured to provide image pixel data suitable for producing a diagnostic image, where the image pixel data is identified from comparisons between the first set of data and the reference set of data and between the reference set of data and the image pixel data

16. The apparatus of claim 14, where the characterization logic (640) is configured to provide diagnostic information, where the diagnostic information is identified from comparisons between the first set of data and the reference set of data and between the reference set of data and the image pixel data.

17. The apparatus of claim 14, where N is greater than two where at least one member of the N sequence blocks (330) differs from at least one other member of the N sequence blocks (330) in at least one of, the number of $\alpha 2$ pulses in a sequence block, the spacing of $\alpha 2$ pulses in a sequence block, the phase of $\alpha 2$ pulses in a sequence block, and the amplitude of $\alpha 2$ pulses in a sequence block.

18. The apparatus of claim 14, wherein the one or more parameters of the material are one or more of, relaxation parameters such as T1 associated with the material or T2 associated with the material, and non-relaxation parameters such as a diffusion coefficient associated with the material, a spin density associated with the material, a proton density associated with the material, a magnetic field to which the material was exposed, a magnetic gradient field to which the material was exposed, a tissue type of the material, and an identification of the material, T1 being spin-lattice relaxation, and T2 being spin-spin relaxation.

19. The apparatus of claim 14, where for the step of comparing the characterization logic (640) is configured to identify one or more of, a portion of the reference NMR signal evolution related to the determined NMR signal evolution (SE), a degree to which a portion of the reference NMR signal evolution is related to the determined NMR signal evolution (SE), and the likelihood that a portion of the reference NMR signal evolution is related to the determined NMR signal evolution (SE).

20. The apparatus of claim 18, where the reference NMR signal evolution includes one or more of, a previously acquired signal evolution, a modeled signal evolution, and a simulated signal evolution.

21. The apparatus of claim 14, where for the step of comparing the characterization logic (640) is configured to compare the determined NMR signal evolution (SE) to the reference NMR signal evolutions using one or more of, pattern matching, selecting, minimizing, and optimizing.

22. The apparatus of claim 21, where pattern matching includes one or more of, orthogonal matching pursuit, categorical sequence labeling, regression, clustering, classification, real valued sequence labeling, parsing, Bayesian methods, Markov methods, ensemble learning methods, and template matching.

23. The apparatus of claim 21, where optimization includes one or more of, least squares optimization, regularized least squares optimization, basis pursuit optimization, and matching pursuit optimization.

**Patentansprüche**

1. Verfahren (400), umfassend:
Steuern eines Kernspinresonanz-(NMR)-Geräts (600), um ein Material einer NMR-Fingerprinting-Anregung auszusetzen, durch:

Steuern des NMR-Geräts
zum Anwenden (410) von Radiofrequenz (RF)-Energie auf das Material in einer Folge von variablen Sequenzblöcken (330), wobei ein Sequenzblock (330) eine Vielzahl von Sequenzblockparametern umfasst und eine oder mehrere Anregungsphasen, eine oder mehrere Auslesephasen und eine oder mehrere Wartephasen umfasst, wobei N der Sequenzblockparameter entweder nichtlinear, zufällig und/oder pseudozufällig im Laufe der Folge von variablen Sequenzblöcken (330) variiert werden, wobei N eine ganze Zahl größer als eins ist, wobei N selbst mindestens einmal während der Folge von variablen Sequenzblöcken (330) variiert wird, wobei das Material eine oder mehrere resonante Spezies (R1; R2) enthält, wobei die während eines Sequenzblocks (330) angewendete RF-Energie so konfiguriert ist, dass sie die eine oder mehrere Resonanzspezies (R1; R2) in dem Material veranlasst, simultan individuelle NMR-Signale (NMR1; NMR2) zu erzeugen, und Steuern der NMR-Vorrichtung zum Erfassen (420) der erzeugten NMR-Signale und zum Bestimmen (430) einer NMR-Signalentwicklung (SE) aus den erfassten NMR-Signalen (NMR1; NMR2), wobei die Signalentwicklung (SE) durch die Folge von variablen Sequenzblöcken (330) bestimmt wird; Bestimmen eines oder mehrerer Parameter des Materials zur Charakterisierung (450) einer Eigenschaft des Materials durch Vergleichen (440) der bestimmten NMR-Signalentwicklung (SE), die den als Reaktion auf die NMR-Fingerprinting-Anregung aus dem Material erfassten NMR-Signalen (NMR1; NMR2) zugeordnet ist, mit Referenz-NMR-Signalentwicklungen, für die der eine oder die mehreren Parameter bekannt sind.

2. Verfahren nach Anspruch 1, wobei es sich bei dem einen oder den mehreren Parametern des Materials um einen oder mehrere von Relaxationsparametern wie mit dem Material assoziierte T1 oder mit dem Material assoziierte T2 und Nicht-Relaxationsparametern wie ein mit dem Material assoziierter Diffusionskoeffizient, eine mit dem Material assoziierte Spindichte, eine mit dem Material assoziierte Protonendichte, ein Magnetfeld, dem das Material ausgesetzt war, ein magnetisches Gradientenfeld, dem das Material ausgesetzt war, ein Gewebetyp des Materials und eine Identifikation des Materials handelt, wobei T1 eine Spin-Gitter-Relaxation und T2 eine Spin-Spin-Relaxation ist.

3. Verfahren nach Anspruch 2, wobei der Schritt des Vergleichens eines oder mehrere umfasst von: Identifizieren eines Teils der Referenz-NMR-Evolutionen, die mit der bestimmten NMR-Signalentwicklung (SE) in Beziehung stehen, Identifizieren eines Grades, zu dem ein Teil der Referenz-NMR-Evolutionen mit der bestimmten NMR-Signalentwicklung (SE) in Beziehung steht, und Identifizieren einer Wahrscheinlichkeit, dass ein Teil der Referenz-NMR-Evolutionen mit der bestimmten NMR-Signalentwicklung (SE) in Beziehung steht.

4. Verfahren nach Anspruch 1, wobei die Referenz-NMR-Signalentwicklung eine oder mehrere der folgenden umfasst: eine zuvor erfasste Signalentwicklung, eine modellierte Signalentwicklung und eine simulierte Signalentwicklung.

5. Verfahren nach Anspruch 1, wobei der Schritt des Vergleichens eines oder mehrere der Folgenden umfasst: Pattern Matching, Auswählen, Minimieren und Optimieren.

6. Verfahren nach Anspruch 5, wobei das Pattern Matching eines oder mehrere der folgenden Verfahren umfasst: orthogonales Matching-Verfahren, kategoriale Sequenzkennzeichnung, Regression, Clustering, Klassifizierung, reellwertige Sequenzkennzeichnung, Parsing, Bayes'sche Verfahren, Markov-Verfahren, Ensemble-Lernverfahren und Template Matching.

7. Verfahren nach Anspruch 5, wobei die Optimierung eines oder mehrere der Folgenden umfasst: Optimierung der kleinsten Quadrate, regulierte Optimierung der kleinsten Quadrate, Basis-Pursuit-Optimierung und Matching-Pursuit-Optimierung.

8. Verfahren nach Anspruch 1, wobei die Sequenzblockparameter Echozeit (TE), Flipwinkel (FA), Phasenkodierung, Diffusionskodierung, Flusskodierung, RF-Pulsamplitude, RF-Pulsphase, Anzahl der RF-Pulse, Typ des magnetischen Gradienten (GS; GR; GP), der zwischen einem Anregungsabschnitt eines Sequenzblocks (330) und einem Ausleseabschnitt eines Sequenzblocks (330) angewendet wird, Anzahl der magnetischen Gradienten (GS; GR; GP), die zwischen einem Anregungsabschnitt eines Sequenzblocks (330) und einem Ausleseabschnitt eines Sequenzblocks (330) angewendet werden, Art des magnetischen Gradienten (GS; GR; GP), der zwischen einem Ausleseabschnitt eines Sequenzblocks (330) und einem Anregungsabschnitt eines Sequenzblocks (330) angewendet wird, Anzahl der magnetischen Gradienten (GS; GR; GP), die zwischen einem Ausleseabschnitt eines Sequenzblocks (330) und einem Anregungsabschnitt eines Sequenzblocks (330) angewendet werden, Art des magnetischen Gradienten (GS; GR; GP), der während eines Ausleseabschnitts eines Sequenzblocks (330) angewandt wird, Anzahl der magnetischen Gradienten (GS; GR; GP), die während eines Ausleseabschnitts eines Sequenzblocks (330) angewandt werden, Ausmaß des RF-Spoilings und Ausmaß des magnetischen Gradienten-Spoilings.

9. Verfahren nach Anspruch 1, umfassend:
Steuern der NMR-Vorrichtung, um eines oder mehrere der Folgenden zu variieren: die Zeitspanne zwischen Sequenzblöcken (330) in der Folge variabler Sequenzblöcke (330), die relative Amplitude von RF-Pulsen in Sequenzblöcken (330) in der Folge variabler Sequenzblöcke (330) und die relative Phase von RF-Pulsen in Sequenzblöcken (330) in der Folge variabler Sequenzblöcke (330).

10. Verfahren nach Anspruch 1, wobei N größer als zwei ist und wobei mindestens ein Prozent der Glieder der Folge von variablen Sequenzblöcken (330) einzigartig sind.

11. Verfahren nach Anspruch 1, wobei die Referenz-NMR-Signalentwicklungen Signalentwicklungen (SE) außerhalb des Satzes von Signalentwicklungen (SE) umfassen, **gekennzeichnet durch**:

$$SE = A - Be^{-t/C}$$

wobei:

SE eine Signalentwicklung ist,
A eine Konstante ist,
B eine Konstante ist,
t die Zeit ist, und
C ein einzelner Relaxationsparameter ist.

12. Verfahren nach Anspruch 1, wobei die Referenz-NMR-Signalentwicklungen Signalentwicklungen (SE) umfassen, die aus einem Satz von Signalen ausgewählt sind, die beschrieben sind durch:

$$SE = \prod_{i=1}^{N_A} \sum_{j=1}^{N_{RF}} R_i(\alpha) R_{RFij}(\alpha,\varphi)\, R(G) E_i(T1,T2,D)$$

wobei:

SE eine Signalentwicklung ist,
$N_A$ eine Anzahl von Sequenzblöcken ist,
$N_{RF}$ die Anzahl der RF-Pulse in einem Sequenzblock ist,
$\alpha$ der Flip-Winkel ist,
$\Phi$ der Phasenwinkel ist,
$Ri(\alpha)$ eine Rotation aufgrund von Off-Resonanz ist,
$R_{RFij}(\alpha, \Phi)$ eine Rotation aufgrund von RF-Differenzen ist,
$R(G)$ eine Rotation aufgrund eines magnetischen Gradienten ist,
T1 die Spin-Gitter-Relaxation ist,
T2 die Spin-Spin-Relaxation ist,
D die Diffusionsrelaxation ist, und
E;(T1, T2, D) mit Magnetisierungsunterschieden verbunden ist.

13. Verfahren nach Anspruch 1, wobei die Referenz-NMR-Signalentwicklungen Signalentwicklungen (SE) umfassen, die aus einem Satz von Signalen ausgewählt sind, die beschrieben sind durch:

$$SE = \prod_{i=1}^{N_A} \prod_{j=1}^{N_{RF}} R_i(\alpha) R_{RFij}(\alpha,\varphi)\, R(G) E_i(T1,T2,...)$$

wobei:

SE eine Signalentwicklung ist,
$N_A$ eine Anzahl von Sequenzblöcken ist,

$N_{RF}$ die Anzahl der RF-Pulse in einem Sequenzblock ist,

$\alpha$ der Flip-Winkel ist,

$\Phi$ der Phasenwinkel ist,

Ri($\alpha$) eine Rotation aufgrund von Off-Resonanz ist,

$R_{RFij}(\alpha, \Phi)$ eine Rotation aufgrund von RF-Differenzen ist,

R(G) eine Rotation aufgrund eines magnetischen Gradienten ist,

T1 die Spin-Gitter-Relaxation ist,

T2 die Spin-Spin-Relaxation ist,

EI(T1, T2, D) mit Magnetisierungsunterschieden verbunden ist.

14. NMR-Vorrichtung (600), umfassend:

eine NMR-Logik (610), die so konfiguriert ist, dass sie erfasste NMR-Signale empfängt, wobei die NMR-Logik (610) so konfiguriert ist, dass sie ein NMR-Gerät so steuert, dass es ein Material einer NMR-Fingerprinting-Anregung aussetzt, durch:

- Steuern des NMR-Gerätes (600), um Radiofrequenz (RF)-Energie auf das Material in einer Folge von variablen Sequenzblöcken (330) anzuwenden, wobei ein Sequenzblock (330) eine Vielzahl von Sequenzblockparametern umfasst und eine oder mehrere Anregungsphasen, eine oder mehrere Auslesephasen und eine oder mehrere Wartephasen umfasst, wobei N der Sequenzblockparameter entweder nichtlinear, zufällig und/oder pseudozufällig im Laufe der Folge von variablen Sequenzblöcken (330) variiert werden, wobei N eine ganze Zahl größer als eins ist,

wobei N selbst mindestens einmal während der Folge von variablen Sequenzblöcken (330) variiert wird, wobei das Material eine oder mehrere resonante Spezies (R1; R2) enthält, wobei die während eines Sequenzblocks (330) angewendete RF-Energie so konfiguriert ist, dass sie die eine oder mehrere Resonanzspezies (R1; R2) in dem Material veranlasst, simultan individuelle NMR-Signale (NMR1; NMR2) zu erzeugen, und

- Steuern des NMR-Gerätes (600), um die erzeugten NMR-Signale (NMR1; NMR2) zu erfassen;

eine Signallogik (620), die so konfiguriert ist, dass sie eine NMR-Signalentwicklung (SE) aus den erfassten NMR-Signalen (NMR1; NMR2) bestimmt, wobei die Signalentwicklung (SE) durch die Folge von variablen Sequenzblöcken (330) bestimmt wird;

eine Charakterisierungslogik (640), die so konfiguriert ist, dass sie einen oder mehrere Parameter des Materials bestimmt, um eine Eigenschaft des Materials zu charakterisieren, indem sie die bestimmte NMR-Signalentwicklung (SE), die mit den als Reaktion auf die NMR-Fingerprinting-Anregung von dem Material erfassten NMR-Signalen (NMR1; NMR2) assoziiert ist, mit Referenz-NMR-Signalentwicklungen vergleicht, für die der eine oder mehrere Parameter bekannt sind.

15. Vorrichtung nach Anspruch 14, wobei die Charakterisierungslogik (640) so konfiguriert ist, dass sie Bildpixeldaten bereitstellt, die zur Erzeugung eines Diagnosebildes geeignet sind, wobei die Bildpixeldaten aus Vergleichen zwischen dem ersten Datensatz und dem Referenzdatensatz und zwischen dem Referenzdatensatz und den Bildpixeldaten identifiziert werden

16. Vorrichtung nach Anspruch 14, wobei die Charakterisierungslogik (640) konfiguriert ist, um Diagnoseinformationen bereitzustellen, wobei die Diagnoseinformationen aus Vergleichen zwischen dem ersten Datensatz und dem Referenzdatensatz und zwischen dem Referenzdatensatz und den Bildpixeldaten identifiziert werden.

17. Vorrichtung nach Anspruch 14, wobei N größer als zwei ist und mindestens ein Glied der N Sequenzblöcke (330) sich von mindestens einem anderen Glied der N Sequenzblöcke (330) in mindestens einem der folgenden Punkte unterscheidet: der Anzahl der $\alpha 2$-Pulse in einem Sequenzblock, dem Abstand der $\alpha 2$-Pulse in einem Sequenzblock, der Phase der $\alpha 2$-Pulse in einem Sequenzblock, und der Amplitude der $\alpha 2$-Pulse in einem Sequenzblock.

18. Vorrichtung nach Anspruch 14, wobei der eine oder die mehreren Parameter des Materials einer oder mehrere ist von Relaxationsparametern wie mit dem Material assoziierte T1 oder mit dem Material assoziierte T2 und Nicht-Relaxationsparametern wie ein mit dem Material assoziierter Diffusionskoeffizient, eine mit dem Material assoziierte Spindichte, eine mit dem Material assoziierte Protonendichte, ein Magnetfeld, dem das Material ausgesetzt war, ein magnetisches Gradientenfeld, dem das Material ausgesetzt war, ein Gewebetyp des Materials und eine Identifikation des Materials sind, wobei T1 eine Spin-Gitter-Relaxation und T2 eine Spin-Spin-Relaxation ist.

19. Vorrichtung nach Anspruch 14, wobei für den Schritt des Vergleichens die Charakterisierungslogik (640) so konfiguriert ist, dass sie eines oder mehrere der folgenden Merkmale identifiziert: einen Teil der NMR-Referenzsignalentwicklung, der mit der bestimmten NMR-Signalentwicklung (SE) in Beziehung steht, einen Grad, zu dem ein Teil der NMR-Referenzsignalentwicklung mit der bestimmten NMR-Signalentwicklung (SE) in Beziehung steht, und die Wahrscheinlichkeit, dass ein Teil der NMR-Referenzsignalentwicklung mit der bestimmten NMR-Signalentwicklung (SE) in Beziehung steht.

20. Vorrichtung nach Anspruch 18, wobei die Referenz-NMR-Signalentwicklung eine oder mehrere der folgenden umfasst: eine zuvor erfasste Signalentwicklung, eine modellierte Signalentwicklung und eine simulierte Signalentwicklung.

21. Vorrichtung nach Anspruch 14, wobei für den Schritt des Vergleichens die Charakterisierungslogik (640) so konfiguriert ist, dass sie die ermittelte NMR-Signalentwicklung (SE) mit den Referenz-NMR-Signalentwicklungen unter Verwendung eines oder mehrerer der folgenden Verfahren vergleicht: Pattern Matching, Auswählen, Minimieren und Optimieren.

22. Vorrichtung nach Anspruch 21, wobei das Pattern Matching eines oder mehrere der folgenden Verfahren umfasst: orthogonaler Matching-Ansatz, kategorische Sequenzkennzeichnung, Regression, Clustering, Klassifizierung, reellwertige Sequenzkennzeichnung, Parsing, Bayes-Verfahren, Markov-Verfahren, Ensemble-Lernverfahren und Template Matching.

23. Vorrichtung nach Anspruch 21, wobei die Optimierung eines oder mehrere der folgenden Verfahren umfasst: Optimierung der kleinsten Quadrate, regulierte Optimierung der kleinsten Quadrate, Basis-Pursuit-Optimierung und Matching-Pursuit-Optimierung.

**Revendications**

1. Méthode (400), comprenant :
la commande d'un appareil (600) de résonance magnétique nucléaire (NMR) pour exposer un matériau à une excitation d'empreinte NMR par :

la commande de l'appareil NMR
pour appliquer (410) une énergie radiofréquence (RF) au matériau dans une série de blocs de séquences (330) variables, dans laquelle un bloc de séquences (330) présente une pluralité de paramètres de bloc de séquences et comprend une ou plusieurs phases d'excitation, une ou plusieurs phases de lecture et une ou plusieurs phases d'attente, dans laquelle N desdits paramètres de bloc de séquences varient de manière non linéaire, aléatoire et/ou pseudo-aléatoire pendant la série de blocs de séquences (330) variables, N étant un nombre entier supérieur à un,

dans laquelle N lui-même varie au moins une fois pendant la série de blocs de séquences (330) variables, dans laquelle le matériau contient une ou plusieurs espèces résonantes (R1 ; R2), dans laquelle l'énergie RF appliquée pendant un bloc de séquence (330) est configurée pour amener les une ou plusieurs espèces résonantes (R1; R2) dans le matériau à produire simultanément des signaux NMR (NMR1 ; NMR2) individuels, et

la commande de l'appareil NMR pour acquérir (420) les signaux NMR produits et pour déterminer (430) une évolution de signal (SE) NMR à partir des signaux NMR (NMR1 ; NMR2) acquis, dans laquelle l'évolution de signal (SE) est déterminée par la série de blocs de séquences (330) variables ; la détermination d'un ou de plusieurs paramètres du matériau pour caractériser (450) une propriété du matériau en comparant (440) l'évolution de signal (SE) NMR déterminée associée aux signaux NMR (NMR1 ; NMR2) acquis à partir du matériau en réponse à l'excitation d'empreinte NMR pour référencer des évolutions de signal NMR pour lesquelles lesdits un ou plusieurs paramètres sont connus.

2. Méthode selon la revendication 1, dans laquelle les un ou plusieurs paramètres du matériau sont un ou plusieurs parmi des paramètres de relaxation tels que T1 associé au matériau ou T2 associé au matériau et des paramètres de non-relaxation tels qu'un coefficient de diffusion associé au matériau, une densité de spin associée au matériau, une densité de proton associée au matériau, un champ magnétique auquel le matériau a été exposé, un champ de gradient magnétique auquel le matériau a été exposé, un type de tissu du matériau, et une identification du matériau, T1 étant la relaxation spin-réseau, et T2 étant la relaxation spin-spin.

3. Méthode selon la revendication 2, dans laquelle l'étape de comparaison comprend un ou plusieurs parmi l'identification d'une partie des évolutions de NMR de référence relatives à l'évolution de signal (SE) NMR déterminée, l'identification d'un degré auquel une partie des évolutions de NMR de référence est associée à l'évolution de signal (SE) NMR déterminée, et l'identification de la probabilité qu'une partie des évolutions de NMR de référence soit associée à l'évolution de signal (SE) NMR déterminée.

4. Méthode selon la revendication 1, où l'évolution de signal NMR de référence comprend un ou plusieurs parmi une évolution de signal précédemment acquise, une évolution de signal modélisée, et une évolution de signal simulée.

5. Méthode selon la revendication 1, où l'étape de comparaison comprend un ou plusieurs parmi un appariement de diagrammes, une sélection, une minimisation, et une optimisation.

6. Méthode selon la revendication 5, où l'appariement de diagrammes comprend un ou plusieurs parmi une recherche d'appariement orthogonal, un étiquetage de séquence catégorielle, une régression, un regroupement, une classification, un étiquetage de séquence à valeur réelle, une analyse, des méthodes bayésiennes, des méthodes de Markov, des méthodes d'apprentissage d'ensemble, et un appariement de modèles.

7. Méthode selon la revendication 5, où l'optimisation comprend un ou plusieurs parmi une optimisation par les moindres carrés, une optimisation par les moindres carrés régularisés, une optimisation par recherche de base, et une optimisation par recherche d'appariement.

8. Méthode selon la revendication 1, où les paramètres de bloc de séquence comprennent un temps d'écho (TE), un angle de bascule (FA), un codage de phase, un codage de diffusion, un codage de flux, une amplitude d'impulsion RF, une phase d'impulsion RF, un nombre d'impulsions RF, un type de gradient magnétique (GS ; GR ; GP) appliqué entre une partie d'excitation d'un bloc de séquence (330) et une partie de lecture d'un bloc de séquence (330), un nombre de gradients magnétiques (GS ; GR ; GP) appliqué entre une partie d'excitation d'un bloc de séquence (330) et une partie de lecture d'un bloc de séquence (330), un type de gradient magnétique (GS ; GR ; GP) appliqué entre une partie de lecture d'un bloc de séquence (330) et une partie d'excitation d'un bloc de séquence (330), un nombre de gradients magnétiques (GS ; GR ; GP) appliqué entre une partie de lecture d'un bloc de séquence (330) et une partie d'excitation d'un bloc de séquence (330), un type de gradient magnétique (GS ; GR ; GP) appliqué pendant une partie de lecture d'un bloc de séquence (330), un nombre de gradients magnétiques (GS ; GR ; GP) appliqué pendant une partie de lecture d'un bloc de séquence (330), une quantité de brouillage de RF, et une quantité de brouillage de gradient magnétique.

9. Méthode selon la revendication 1, comprenant :
la commande de l'appareil NMR pour faire varier un ou plusieurs parmi la quantité de temps entre des blocs de séquences (330) dans la série de blocs de séquences (330) variables, l'amplitude relative des impulsions RF dans des blocs de séquences (330) dans la série de blocs de séquences (330) variables, et la phase relative des impulsions RF dans des blocs de séquences (330) dans la série de blocs de séquences (330) variables.

10. Méthode selon la revendication 1, où N est supérieur à deux et où au moins un pour cent des membres de la série de blocs de séquences (330) variables est unique.

11. Méthode selon la revendication 1, où les évolutions de NMR de référence comprennent les évolutions de signal (SE) en dehors de l'ensemble d'évolutions de signal (SE) **caractérisées par** :

$$SE = A - Be^{-t/c}$$

où :

SE est une évolution de signal,

A est une constante,
B est une constante,
t est un temps, et
C est un unique paramètre de relaxation.

**12.** Méthode selon la revendication 1, où les évolutions de NMR de référence comprennent des évolutions de signal (SE) sélectionnées parmi un ensemble de signaux décrits par :

$$SE = \prod_{i=1}^{N_A} \sum_{j=1}^{N_{RF}} R_i(\alpha) R_{RFij}(\alpha, \varphi)\, R(G) E_i(T1, T2, D)$$

où :

SE est une évolution de signal,
$N_A$ est un nombre de blocs de séquences,
$N_{RF}$ est un nombre d'impulsions RF dans un bloc de séquences,
$\alpha$ est un angle de bascule,
$\Phi$ est un angle de phase,
$R_i(\alpha)$ est une rotation due à une hors résonance,
$R_{RFij}(\alpha, \Phi)$ est une rotation due aux différences RF,
$R(G)$ est une rotation due à un gradient magnétique,
T1 est la relaxation spin-réseau,
T2 est la relaxation spin-spin,
D est la relaxation par diffusion, et
$E_i(T1, T2, D)$ est associé aux différences d'aimantation.

**13.** Méthode selon la revendication 1, où les évolutions de NMR de référence comprennent des évolutions de signal (SE) sélectionnées parmi un ensemble de signaux décrits par :

$$SE = \prod_{i=1}^{N_A} \prod_{j=1}^{N_{RF}} R_i(\alpha) R_{RFij}(\alpha, \varphi)\, R(G) E_i(T1, T2, \ldots)$$

où :

SE est une évolution de signal,
$N_A$ est un nombre de blocs de séquences,
$N_{RF}$ est un nombre d'impulsions RF dans un bloc de séquences,
$\alpha$ est un angle de bascule,
$\Phi$ est un angle de phase,
$R_i(\alpha)$ est une rotation due à une hors résonance,
$R_{RFij}(\alpha, \Phi)$ est une rotation due aux différences RF,
$R(G)$ est une rotation due à un gradient magnétique,
T1 est la relaxation spin-réseau,
T2 est la relaxation spin-spin, et
$E1(T1, T2, \ldots)$ est associé aux changements d'aimantation.

**14.** Appareil NMR (600), comprenant :

une logique NMR (610) configurée pour recevoir des signaux NMR acquis, dans lequel la logique NMR (610) est configurée pour commander un appareil NMR pour exposer un matériau à une excitation d'empreinte NMR par :

- la commande de l'appareil NMR (600) pour appliquer une énergie radiofréquence (RF) au matériau dans une série de blocs de séquences (330) variables, dans lequel un bloc de séquences (330) présente une pluralité de paramètres de bloc de séquence et comprend une ou plusieurs phases d'excitation, une ou plusieurs phases de lecture et une ou plusieurs phases d'attente, dans lequel N desdits paramètres de bloc de séquences varient de manière non linéaire, aléatoire et/ou pseudo-aléatoire pendant la série de blocs de séquences (330) variables, N étant un nombre entier supérieur à un,

dans lequel N lui-même varie au moins une fois pendant la série de blocs de séquences (330) variables, dans lequel le matériau contient une ou plusieurs espèces résonantes (R1 ; R2), dans lequel l'énergie RF appliquée pendant un bloc de séquences (330) est configurée pour amener les une ou plusieurs espèces résonantes (R1; R2) dans le matériau à produire simultanément des signaux NMR (NMR1 ; NMR2) individuels, et

- la commande de l'appareil NMR (600) pour acquérir les signaux NMR (NMR1 ; NMR2) produits ;

une logique de signal (620) configurée pour déterminer une évolution de signal (SE) NMR à partir des signaux NMR (NMR1 ; NMR2) acquis, dans lequel l'évolution de signal (SE) est déterminée par la série de blocs de séquences (330) variables ;
une logique de caractérisation (640) configurée pour déterminer un ou plusieurs paramètres du matériau pour caractériser une propriété du matériau en comparant l'évolution de signal (SE) NMR déterminée associée aux signaux NMR (NMR1 ; NMR2) acquis à partir du matériau en réponse à l'excitation d'empreinte NMR pour référencer des évolutions de signal NMR pour lesquelles lesdits un ou plusieurs paramètres sont connus.

15. Appareil selon la revendication 14, où la logique de caractérisation (640) est configurée pour fournir des données de pixels d'image appropriées pour produire une image de diagnostic, où les données de pixels d'image sont identifiées à partir de comparaisons entre le premier ensemble de données et l'ensemble de données de référence et entre l'ensemble de données de référence et les données de pixels d'image

16. Appareil selon la revendication 14, où la logique de caractérisation (640) est configurée pour fournir des informations de diagnostic, où les informations de diagnostic sont identifiées à partir de comparaisons entre le premier ensemble de données et l'ensemble de données de référence et entre l'ensemble de données de référence et les données de pixels d'image.

17. Appareil selon la revendication 14, où N est supérieur à deux, où au moins un membre des N blocs de séquences (330) diffère d'au moins un autre membre des N blocs de séquences (330) dans au moins un parmi le nombre d'impulsions $\alpha2$ dans un bloc de séquences, l'espacement des impulsions $\alpha2$ dans un bloc de séquences, la phase des impulsions $\alpha2$ dans un bloc de séquences, et l'amplitude des impulsions $\alpha2$ dans un bloc de séquence.

18. Appareil selon la revendication 14, dans lequel les un ou plusieurs paramètres du matériau sont un ou plusieurs parmi des paramètres de relaxation tels que T1 associé au matériau ou T2 associé au matériau, et des paramètres de non-relaxation tels qu'un coefficient de diffusion associé au matériau, une densité de spin associée au matériau, une densité de proton associée au matériau, un champ magnétique auquel le matériau a été exposé, un champ de gradient magnétique auquel le matériau a été exposé, un type de tissu du matériau, et une identification du matériau, T1 étant la relaxation spin-réseau, et T2 étant la relaxation spin-spin.

19. Appareil selon la revendication 14, où pour l'étape de comparaison, la logique de caractérisation (640) est configurée pour identifier un ou plusieurs parmi une partie de l'évolution de signal NMR de référence relative à l'évolution de signal (SE) NMR déterminée, un degré auquel une partie de l'évolution de signal NMR de référence est associée à l'évolution de signal (SE) NMR déterminée, et la probabilité qu'une partie de l'évolution de signal NMR de référence soit associée à l'évolution de signal (SE) NMR déterminée.

20. Appareil selon la revendication 18, où l'évolution de signal NMR de référence comprend un ou plusieurs parmi une évolution de signal précédemment acquise, une évolution de signal modélisée, et une évolution de signal simulée.

21. Appareil selon la revendication 14, où pour l'étape de comparaison, la logique de caractérisation (640) est configurée pour comparer l'évolution de signal (SE) NMR déterminée aux évolutions de signal NMR de référence en utilisant un ou plusieurs parmi un appariement de diagrammes, une sélection, une minimisation, et une optimisation.

**22.** Appareil selon la revendication 21, où l'appariement de diagrammes comprend un ou plusieurs parmi une recherche d'appariement orthogonal, un étiquetage de séquence catégorielle, une régression, un regroupement, une classification, un étiquetage de séquence à valeur réelle, une analyse, des méthodes bayésiennes, des méthodes de Markov, des méthodes d'apprentissage d'ensemble, et un appariement de modèles.

**23.** Appareil selon la revendication 21, où l'optimisation comprend un ou plusieurs parmi une optimisation par les moindres carrés, une optimisation par les moindres carrés régularisés, une optimisation par recherche de base, et une optimisation par recherche d'appariement.

Figure 1

Figure 2

Figure 3

400

Start

Control NMR Apparatus
To Apply RF Energy                    410

Control NMR Apparatus
To Acquire
Simultaneously Produced
NMR Signals                           420

Determine Signal Evolution            430

Compare                               440

End

Figure 4

400

```
                    ┌─────────┐
                    │  Start  │
                    └─────────┘
                         │
                         ▼
          ┌──────────────────────────┐      410
          │  Control NMR Apparatus   │
          │   To Apply RF Energy     │
          └──────────────────────────┘
                         │
                         ▼
          ┌──────────────────────────┐      412
          │        Vary Time,        │
          │ Relative Amp, Relative Phase │
          └──────────────────────────┘
                         │
                         ▼
          ┌──────────────────────────┐      414
          │        Configure         │
          │     Sequence Block       │
          └──────────────────────────┘
                         │
                         ▼
          ┌──────────────────────────┐      416
          │        Configure         │
          │  Later Based on Earlier  │
          └──────────────────────────┘
                         │
                         ▼
          ┌──────────────────────────┐      420
          │  Control NMR Apparatus   │
          │       To Acquire         │
          │  Simultaneously Produced │
          │      NMR Signals         │
          └──────────────────────────┘
                         │
                         ▼
          ┌──────────────────────────┐      430
          │ Determine Signal Evolution │
          └──────────────────────────┘
                         │
                         ▼
          ┌──────────────────────────┐      440
          │         Compare          │
          └──────────────────────────┘
                         │
                         ▼
          ┌──────────────────────────┐      450
          │ Characterize Resonant Species │
          └──────────────────────────┘
                         │
                         ▼
                    ┌─────────┐
                    │   End   │
                    └─────────┘
```

Figure 5

NMR Apparatus <u>600</u>

| NMR Logic<br><u>610</u> | Signal Logic<br><u>620</u> | Matching Logic<br><u>630</u> |

Figure 6

NMR Apparatus 600

| NMR Logic | Signal Logic | Matching Logic |
| --- | --- | --- |
| 610 | 620 | 630 |

Characterization Logic 640

Figure 7

800

| Basic Field Magnet 810 | | Basic Field Magnet Supply 820 |
|---|---|---|

830

| | | Gradient Coils Supply 840 |
|---|---|---|

| RF Antennas 850 | | RF Tx Units 860 |
|---|---|---|

| Basic Field Magnet 810 |
|---|

| Control Computer 870 |
|---|

| Image Computer 880 |
|---|

| Fingerprinting Apparatus 899 |
|---|

| Display 890 |
|---|

Figure 8

Figure 9

Figure 10a

Figure 10b

Figure 11a

Figure 11b

Figure 11c

Figure 12a

Figure 12b

Figure 12c

Figure 12d

NMR Apparatus 1350

Apparatus 1300

First Logic
1310

Signal Logic
1320

Characterization
Logic
1340

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **TWIEG.** Parsing local signal evolution directly from a single-shot MRI signal: a new approach for fMRI. *Magn Reson Med,* November 2003, vol. 50 (5), 1043-52
- **DONEVA et al.** Compressed sensing reconstruction for magnetic resonance parameter mapping. *Magnetic Resonance in Medicine,* October 2010, vol. 64 (4), 1114-1120
- **JAMES S MCKENZIE et al.** Analysis of complex mixtures using high-resolution nuclear magnetic resonance spectroscopy and chemometrics. *Progress in Nuclear Magnetic Resonance Spectroscopy,* 27 April 2011, vol. 59 (4
- **DAN MA et al.** MR Fingerprinting (MRF): a Novel Quantitative Approach to MRI. *Proceedings of the International Society for Magnetic Resonance in Medicine,* 2012, vol. 20, 288